(19) **Europäisches Patentamt European Patent Office Office européen des brevets**

(11) **EP 1 745 290 B1**

(12) **FASCICULE DE BREVET EUROPEEN**

(45) Date de publication et mention de la délivrance du brevet:
**14.08.2013 Bulletin 2013/33**

(21) Numéro de dépôt: **05767635.5**

(22) Date de dépôt: **03.05.2005**

(51) Int Cl.:
*G01N 33/543* (2006.01)    *G01N 33/80* (2006.01)

(86) Numéro de dépôt international:
**PCT/FR2005/001101**

(87) Numéro de publication internationale:
**WO 2005/121805 (22.12.2005 Gazette 2005/51)**

(54) **UTILISATION DE FERROFLUIDES POUR LE PHENOTYPAGE SANGUIN ET APPLICATIONS DERIVEES**

VERWENDUNG VON FERROFLUIDEN ZUR BESTIMMUNG DES BLUTGRUPPENPHENOTYPS UND ABGELEITETE ANWENDUNGEN

USE OF FERROFLUIDS FOR PHENOTYPING BLOOD AND RELATED APPLICATIONS

(84) Etats contractants désignés:
**AT BE BG CH CY CZ DE DK EE ES FI FR GB GR HU IE IS IT LI LT LU MC NL PL PT RO SE SI SK TR**

(30) Priorité: **05.05.2004 FR 0404853**

(43) Date de publication de la demande:
**24.01.2007 Bulletin 2007/04**

(73) Titulaire: **Diagast**
**59120 Loos (FR)**

(72) Inventeurs:
• **BARBREAU, Yves**
**F-59420 Mouvaux (FR)**
• **BOULET, Olivier**
**F-62113 Sailly Labourse (FR)**
• **BOULET, Arnaud**
**F-59133 Camphin en Carembault (FR)**
• **DELANOE, Alexis**
**F-59000 Lille (FR)**
• **FAUCONNIER, Laurence**
**F-59491 Villeneuve D'Ascq (FR)**
• **PELOSIN, Jean-Marc**
**F-59130 Lambersart (FR)**
• **SOUFFLET, Laurent**
**F-59230 Saint Amand les Eaux (FR)**

(74) Mandataire: **Regimbeau**
**20, rue de Chazelles**
**75847 Paris Cedex 17 (FR)**

(56) Documents cités:
EP-A- 0 230 768      EP-A- 0 348 191
EP-A1- 0 194 212     EP-A1- 0 528 708
EP-A2- 0 351 857     EP-A2- 0 755 719
WO-A-02/40159        WO-A1-02/46758
WO-A1-02/46773       WO-A1-98/02752
FR-A1- 2 461 521

**Description**

La présente invention concerne un procédé de phénotypage de groupe sanguin mettant en oeuvre une solution aqueuse de ferrofluide obtenu à partir d'un mélange polyoxoanions de Fe(III) et d'au moins un métal M(II) de degré II d'oxydation. L'invention comprend également un dispositif et un kit permettant de réaliser un tel procédé.

La transfusion sanguine consiste de nos jours à administrer par voie intraveineuse des préparations de concentré de globules rouges (concentrés globulaires) obtenues à partir de sang de donneurs.

Lors d'une transfusion sanguine, le risque premier est lié à la possibilité de réunir dans l'organisme du receveur (la personne transfusée) un anticorps et son antigène érythrocytaire. On trouve en effet à la surface des érythrocytes, appelés encore globules rouges ou hématies, des antigènes membranaires érythrocytaires, notamment des antigènes de groupe (ou système) sanguin, capables d'être reconnus par le système immunitaire et de déclencher une réponse immune.

Les globules rouges du donneur sont dits compatibles avec le sang du receveur si le receveur ne présente pas d'anticorps circulants dirigés contre un antigène érythrocytaire du donneur.

Parmi l'ensemble des variants antigéniques d'un antigène membranaire érythrocytaire constituant les groupes sanguins, plus de vingt systèmes antigéniques érythrocytaires chez l'Homme ont été à ce jour identifiés : système ABO avec les antigènes A ou B, système Rhésus avec l'antigène D, E ou e et C ou c, système Kell avec l'antigène K ou k, Duffy (Fya, Fyb), Kidd (Jka, Jkb) ou encore d'autres systèmes moins fréquemment recherchés en pratique existant aussi tels que MNS, Lewis, etc.. Les individus présentant une même association d'antigènes érythrocytaires appartiennent à un même groupe sanguin érythrocytaire. Les groupes sanguins sont d'autant plus complexes et nombreux que l'on utilise plusieurs systèmes antigéniques.

En dehors de situation pathologique, telle que dans le cas de maladie auto-immune, le sérum d'un individu peut contenir deux types d'anticorps dirigés contre des antigènes érythrocytaires :

-    (i) les anticorps dits réguliers et dirigés contre les antigènes du système ABO (par exemple anticorps anti-A chez l'individu de groupe B). Il s'agit d'immunoglobulines de type IgM qui sont capables d'agglutiner les globules rouges in vitro. Ce phénomène est mis à profit pour déterminer le groupe ABO d'un individu par les épreuves de Beth-Vincent et Simonin, l'épreuve de Beth-Vincent permettant de déterminer les antigènes portés par ses globules rouges (phénotype antigénique) et l'épreuve de Simonin permettant de réaliser l'étude complémentaire, c'est-à-dire de déterminer les anticorps anti-A et/ou anti-B circulants présents dans le sérum de l'individu.

Dans l'épreuve de Beth-Vincent, les globules rouges de l'individu sont mis en présence de sérums tests, ou anticorps tests, possédant chacun un type d'anticorps précis, dirigé contre un antigène du système ABO. Il s'agit donc d'un test d'agglutination des globules rouges avec des sérums tests.

Dans l'épreuve de Simonin, appelée encore contre-épreuve, le sérum de l'individu, contenant ses anticorps circulants, est mis en présence de globules rouges tests, ou érythrocytes tests, appartenant chacun à un groupe antigénique précis du système ABO. Il s'agit donc d'un test d'agglutination du sérum avec des globules rouges tests :

-    (ii) les anticorps dits irréguliers (ou immuns) dont la présence dans le sérum ou plasma est facultative et qui sont dirigés contre des antigènes des systèmes non ABO. Il s'agit le plus souvent d'IgG, apparaissant à l'occasion d'une stimulation antigénique par des globules rouges étrangers, par exemple suite à une immunisation contre un ou plusieurs antigènes lors d'une transfusion sanguine ou encore lors d'une grossesse par réaction immunitaire maternelle dirigée contre les antigènes érythrocytaires foetaux n'appartenant pas au groupe sanguin maternel, notamment lors de l'accouchement.

La recherche de ces anticorps dits irréguliers est appelée Recherche d'Agglutinine Irrégulière (RAI). Ce test est utilisé pour détecter la présence ou non dans le sang d'un individu d'IgG dirigées contre divers antigènes érythrocytaires. Pour cela, on cherche à mettre en évidence la fixation de ces IgG sur des globules rouges tests dont les antigènes sont connus. On procède en parallèle sur de nombreux types de globules rouges, la comparaison des résultats permettant de déduire la ou les IgG présentes.

Le risque est plus important pour les antigènes les plus immunogènes comme le rhésus D mais aussi les autres rhésus (E>c>e>C), le Kell (K), le Duffy (Fy a, Fy b), le Kidd (Jka, Jk b), etc..

En pratique, on ne peut tenir compte de tous ces antigènes pour réaliser une transfusion faute de quoi on ne disposerait jamais du bon groupe sanguin au bon moment, d'autant moins que certaines associations antigéniques sont très rares. La transfusion standard ne tient compte que du groupe dans le système ABO plus le Rhésus D (Rh+ ou Rh-). Dans les situations à risque d'apparition d'une agglutinine irrégulière, on tient compte d'un certain nombre d'autres systèmes, notamment les Rhésus C et E et le Kell, voire encore d'autres systèmes. Il s'agira alors pour ces situations à risques de respecter la compatibilité du groupe sanguin du donneur avec celui du sang du receveur en tenant compte de la présence ou du risque d'apparition de ces agglutinines irrégulières.

Ainsi, chez un patient receveur porteur d'anticorps irréguliers anti-érythrocytaires ou dans une situation à risque comme notamment chez les patients polytransfusés ne possédant pas d'anticorps irréguliers anti-érythrocytaires et chez la femme enceinte, il est indispensable de sélectionner les unités de concentrés érythrocytaires qui vont lui être transfusées de telle sorte que les globules rouges du donneur devront être dépourvus des antigènes contre lesquels les anticorps du receveur sont dirigés ou susceptibles d'apparaître. Cette épreuve de compatibilité est obligatoire chez ces patients et de manière préventive chez tous les receveurs avant l'administration des concentrés érythrocytaires en effectuant une épreuve de compatibilité directe avec les hématies du donneur en présence du sérum ou plasma du receveur. Aucune réaction d'agglutination et/ou de lyse dans les techniques utilisées pour la RAI ne doit être observée.

En pratique clinique transfusionnelle, le phénotypage érythrocytaire, qui correspond à la recherche et à l'identification des antigènes de groupe sanguin à la surface des hématies (à l'exception du système particulier ABO où l'on recherche en outre la présence des anticorps réguliers correspondants), concerne aussi bien le receveur que le donneur.

A l'échelon du receveur et du donneur, trois niveaux de phénotype érythrocytaire existent afin de mettre à disposition du receveur des concentrés érythrocytaires compatibles en fonction des situations à risque :

- la détermination du phénotype groupage ABO (ou groupage ABO) et Rhésus standard (présence ou absence de l'antigène D) ;
- la détermination du phénotype Rhésus Kell (présence ou absence des antigènes C, E, c, e et K) ; et
- la détermination du phénotype étendu (ou élargi) (présence ou absence des antigènes du système Duffy, Fy a et Fy b, du Système Kidd, Jk a et Jk b, et du système MNSs (antigènes S et s), d'autres antigènes pouvant être en outre recherchés selon la nature du risque et/ou de l'anticorps irrégulier mis en évidence dans le sérum du receveur.

Les techniques habituellement utilisées, mises en oeuvre pour rechercher et identifier la présence ou l'absence d'antigène de groupe sanguin à la surface d'érythrocyte de donneur et/ou de receveur, ou visant à rechercher et identifier la présence ou l'absence d'anticorps anti-antigène de groupe sanguin, régulier (pour le groupage ABO) ou irrégulier dans le cadre de la RAI dans le sérum ou plasma de donneur et/ou de receveur, sont bien connues de l'homme de l'art et ne seront ici pas décrites.

Pour le phénotypage, elles consistent en général à rechercher à l'aide de sérums-tests contenant les anticorps appropriés la présence ou l'absence de l'antigène recherché. De préférence, ces anticorps contenus dans ces sérums tests sont de nature agglutinante (IgM ou IgA), ce qui permet d'obtenir une agglutination totale ou partielle des érythrocytes à phénotyper lorsque ces derniers portent l'antigène correspondant à l'anticorps présent dans le sérum test. Néanmoins, il est possible d'utiliser des anticorps tests non agglutinants (de type IgG), l'agglutination étant provoquée au moyen d'une anti-immunoglobuline (technique dite de Coombs indirect) ou la présence d'anticorps tests non agglutinants fixés sur le globule rouge étant visualisée au moyen d'une anti-immunoglobuline fixée sur une phase solide. La lecture de l'agglutination ou des globules rouges sensibilisés avec l'anticorps test fixés sur la phase solide par l'intermédiaire de l'anti-immunoglobuline (Technique dite d'immunoadhérence), pourra être réalisée à l'oeil ou au moyen de lecteur approprié.

Pour la recherche et l'identification dans l'échantillon de sérum ou plasma du patient à tester d'anticorps anti-antigène de groupe sanguin, réguliers pour le groupage ABO ou irréguliers dans le cadre de la RAI, on met en général en présence du sérum ou du plasma du patient avec des érythrocytes-tests (appelés encore hématies-, ou globules rouges-tests, d'antigénicité connue dans un certain nombre de systèmes de groupes sanguins (ABO, Rhésus, Kell, Duffy, Kidd, MNSs, ...). Pour la RAI, pour laquelle les anticorps susceptibles d'être présents sont plutôt de type non agglutinants, les techniques utilisées sont de type Coombs indirect par agglutination au moyen d'une anti-immunoglobuline ou par immunoadhérence sur une phase solide revêtue d'une anti-immunoglobuline.

Pour la RAI, on utilise dans une première étape un panel de globules rouges dit de dépistage (deux ou trois globules rouges de groupe différent choisis de façon à comporter le maximum d'antigènes) permettant de détecter (mais pas d'identifier) le présence ou l'absence d'anticorps irréguliers. Lorsque le dépistage est positif, on réalise alors l'identification de la spécificité du ou des anticorps irréguliers présents au moyen d'au moins un panel de globules rouges dit d'identification comportant en général 10 globules rouges différents phénotypés dans la grande majorité des systèmes de groupes sanguins connus.

On peut noter également que lors de l'épreuve de compatibilité où les hématies du donneur sont mises en présence du sérum du receveur, on effectue en général dans une première étape de centrifugation afin d'observer la présence éventuelle d'une agglutination liée à la présence dans le sérum du receveur d'anticorps agglutinants les globules rouges du donneur (incompatibilité de type ABO ou anticorps irréguliers de type IgM ou IgA), cette première étape étant suivie, si pas d'agglutination, d'une recherche d'anticorps au moyen d'une anti-immunoglobuline (technique de Coombs indirect à l'anti-globuline).

Il existe un grande nombre de variantes des techniques utilisées pour le phénotypage ou la RAI dans le domaine de la transfusion sanguine, ces techniques pouvant être manuelles, sur plaque d'opaline, en tube ou en cupule de microplaque, ou complètement automatisées à l'aide de robot distributeur d'échantillon et de réactif, agitateur, incubateur et lecteur automatique dont les programmes sont adaptés aux techniques mises en oeuvre.

Parmi les techniques utilisées, on peut citer les techniques pour lesquelles la présence d'anticorps anti-antigène de groupe sanguin ou d'antigène de groupe sanguin est basée sur la mise en évidence d'une agglutination de globules rouges après centrifugation mettant en oeuvre une mini colonne de filtration transparente (gel type « sephadex® » ou microbilles) dont l'évasement à l'extrémité supérieure sert de chambre d'incubation et dont le seuil de coupure choisi pour la colonne empêche les hématies agglutinées après centrifugation de traverser la colonne (voir notamment le document brevet EP 0 194 212 ou brevet EP 0 755 719).

On peut également citer les techniques pour lesquelles le phénotypage ou la RAI est basée sur la mise en évidence de globules rouges sensibilisés avec un anticorps après centrifugation puis immunoadhérence mettant en oeuvre une barrière de séparation constituée d'un gel ou d'un liquide dont la densité est choisie de telle manière que seuls les globules rouges peuvent traverser cette barrière lors d'une centrifugation, le container à réaction étant revêtu dans sa partie inférieure d'une anti-immunoglobuline afin de piéger les globules rouges sensibilisés et donner une image caractéristique. Parmi ces techniques, on peut citer le document brevet EP 0 058 780 qui décrit un procédé de phénotypage sanguin dans lequel le mélange réactionnel est centrifugé au travers d'un coussin de densité supérieure (de type solution d'albumine bovine ou de polyvinyl pyrrolidone), ce qui présente l'avantage de supprimer l'étape de lavage des hématies sensibilisées. On peut également citer le document brevet WO 98/02752 qui décrit un procédé général pour déterminer la présence d'un antigène sanguin présent sur des érythrocytes ou d'un anticorps fixant un tel antigène. Dans ce procédé, les érythrocytes sensibilisés ou non, sont séparés des anticorps non fixés par centrifugation grâce à un milieu de séparation dont la densité est supérieure à celle du liquide contenant les anticorps, mais inférieure à celle des érythrocytes, les érythrocytes sensibilisés étant séparés des érythrocytes non sensibilisés sur la paroi inférieure du container à réaction sur lequel est immobilisée une anti-immunoglobuline, les érythrocytes non sensibilisés étant rassemblés dans le fond du container, l'analyse de l'image finale obtenue étant spécifique de la présence ou non de l'analyte recherché.

Parmi les variantes des techniques utilisées pour le phénotypage ou la RAI, on peut également citer celles qui ont été développées de manière générale pour la recherche dans un échantillon d'un analyte capable de se fixer sur une cellule en utilisant des particules magnétiques, ceci notamment afin de supprimer la centrifugation, opération nécessaire notamment dans les techniques basées sur l'agglutination comme la technique à l'anti-globuline (Coombs indirect par agglutination ou par immunoadhérence sur phase solide) pour la RAI ou encore pour le phénotypage, ou encore, comme pour la RAI, lorsqu'il est nécessaire de laver les globules rouges sensibilisés afin d'éliminer les anticorps non spécifiques capables de reconnaître l'anti-immunoglobuline utilisée à l'étape suivante.

En effet, l'étape de centrifugation est toujours difficile à mettre en oeuvre dans les procédés que l'on souhaite entièrement automatiser, notamment due au coût et à l'encombrement des centrifugeuses, leur manipulation, etc..

Les particules magnétiques sont depuis longtemps utilisées pour la détection de complexes de type ligand-récepteur ou anticorps-antigène, on peut citer par exemple les procédés tels que ceux décrits dans les documents brevets suivants :

- le document WO 92/17781 qui décrit une méthode de détermination de la présence d'un ligand dans un échantillon dans laquelle on incube des particules de latex magnétiques, pouvant être de couleurs différentes, revêtues d'une substance, telle un anticorps, capable de se fixer avec ce ligand, puis on applique un champ magnétique au milieu d'incubation et enfin on observe la présence ou l'absence d'agglutination ; ou encore
- le document EP 0 426 170 qui décrit une méthode de détermination de la présence d'un ligand dans un échantillon, méthode dans laquelle on incube des particules de gélatine magnétiques sensibilisées avec des antigènes ou des anticorps capables de se fixer avec ce ligand, puis on applique un champ magnétique au milieu d'incubation et enfin on observe la présence ou l'absence d'agglutination, ladite méthode étant caractérisée en ce qu'on observe l'état de glissement de ces particules après inclinaison du container, notamment une cupule de microplaque à fond en V.

De telles particules magnétiques ont déjà été utilisées en immunohématologie pour le phénotypage et/ou la RAI. Parmi les documents décrivant de telles applications, on peut citer notamment :

- le document EP 0 351 857 qui décrit un procédé de dosage immunologique utilisant des marqueurs magnétisés tels que des anticorps ou des antigènes fixés sur des billes de latex magnétiques. Ces marqueurs sont capables de se lier à une substance que l'on souhaite déterminer dans une étape d'immunoréaction, et l'on rassemble ensuite les particules magnétiques à marqueur sur une région prédéterminée de la surface d'une paroi dans un récipient de mesure à l'aide d'un aimant positionné sous cette cupule et sous l'action d'un champ magnétique, ce procédé pouvant comprendre une substance qui peut se lier spécifiquement à la substance à déterminer immobilisée sur une région prédéterminée de la surface de la paroi du récipient de mesure. Il est décrit notamment une technique RAI par immunoadhérence dans laquelle des érythrocytes préalablement fixés au fond d'une cupule de microplaque sont ensuite sensibilisés avec le sérum du receveur, puis lavés (par aspiration et injection du liquide de lavage), puis dans laquelle cupule on ajoute les billes de latex magnétiques revêtues d'une anti-immunoglobuline avant d'appliquer un champ magnétique ;

- le document EP 0 528 708 qui décrit un procédé de détection par immunoadhérence d'une substance biologique susceptible d'être présente dans un échantillon. Dans ce procédé, les érythrocytes à phénotyper ou servant de panel de dépistage et/ou d'identification sont fixés préalablement sur le fond d'une microplaque. Après sensibilisation des érythrocytes ainsi fixés avec le sérum test (pour le phénotypage) ou le sérum du receveur à tester (pour la RAI), les cupules sont lavées et on ajoute ensuite des billes de latex magnétiques revêtues d'anti-immunoglobuline. Dans ce procédé, on applique notamment deux types de champ magnétique successifs (de type vertical et circulaire) afin de déplacer les particules magnétiques non liées spécifiquement avec la substance à rechercher ; et

- le document brevet EP 0 230 768 qui décrit un procédé de co-agrégation de particules magnétiques capable de se lier à une substance contenue dans un échantillon au moyen de composés polycationiques ou polyanioniques en présence d'un champ magnétique. Ce document décrit en particulier la séparation du plasma d'un échantillon de sang total contenant des globules rouges dans lequel procédé, on ajoute séquentiellement dans un container placé sur un aimant l'échantillon de sang total et un ferrofluide ($FeCl_2$/$FeCl_3$) revêtu d'albumine bovine sérique succinylée, les agrégats de particules d'érythrocytes ainsi obtenus étant entraînés vers l'aimant permettant ainsi de récupérer le plasma clarifié par décantation. Dans ce document, il est également décrit un procédé permettant de quantifier la présence d'un anticorps anti-RH (anti-D) dans un échantillon de plasma préparé selon le procédé précédent lequel est incubé en présence d'une suspension de globules rouges RH+ fluorescents et auquel mélange on ajoute ensuite et séquentiellement le ferrofluide succinylé et du polybrène, les globules rouges étant lavés plusieurs fois par application d'un champ magnétique et décantation avant l'ajout d'une anti-immunoglobuline. La quantification de l'anticorps anti-RH dans l'échantillon de plasma est évaluée par comparaison à des témoins en analysant les fluctuations de quantité de fluorescence observée dans un volume donné.

- le document brevet EP 0 348 191 qui décrit un procédé de détermination de la présence d'un ligand lié à la surface d'un premier type de particules dans un milieu liquide. Ce procédé comprend la fourniture, en combinaison, d'un milieu liquide susceptible de contenir un ligand lié à la surface d'un premier type de particules, de moyens pour agglutiner les particules en relation avec la présence dudit ligand, d'un second type de particules possédant un ligand identique ou différent du premier, et de moyens pour agglutiner le second type de particules. L'agglutination du premier et du second type de particules est détectable distinctement par mesure spectroscopique. Le milieu est incubé et l'agglutination de chacune des particules est déterminée spectroscopiquement sans séparer les premier et le second types de particules.

- le document WO 02/40159 qui décrit un dispositif permettant d'appliquer un champ magnétique à une plaque de microtitration, ledit dispositif comprenant un substrat et une pluralité d'éléments magnétiques disposés sur ledit substrat, lesdits éléments magnétiques étant rangés en parallèle les uns des autres de façon à ce que l'axe longitudinal de chaque élément magnétique est approximativement centré sous une rangée ou une colonne de puits lorsque la plaque est en place.

- le document WO 02/46758 qui décrit un procédé de magnétisation de marqueurs chimiques ou biologiques à l'aide de particules magnétiques. Ce procédé comprend les étapes consistant à : 1) activer les particules magnétiques de sorte à modifier leur état de surface, 2) mettre en contact les particules magnétiques activées avec les marqueurs, de sorte à créer des liaisons non spécifiques entre eux. Lesdits marqueurs activés peuvent être utilisés comme réactifs ou analytes dans un test d'analyse biologique.

Si ces dernières techniques mettant en oeuvre des billes magnétiques revêtues d'anti-immunoglobuline ou un ferrofluide modifié permettent de s'affranchir des étapes de centrifugation, elles nécessitent l'utilisation de phases solides préalablement revêtues de globules tests ou à phénotyper, peu stables ou devant être préparées par l'utilisateur final. D'autre part, voir notamment le document EP 0 230 768, l'utilisation de certaines compositions magnétiques provoque la co-agrégation non spécifique des globules rouges après application du champ magnétique ce qui abolit définitivement la possibilité d'une lecture d'une agglutination spécifique des globules rouges en présence de l'anticorps anti-antigène érythrocytaire, technique de référence dans le domaine de la transfusion sanguine.

Ainsi, il reste de pouvoir disposer d'un procédé rapide et simple, pouvant être mis en oeuvre sur un support pratique et disponible tel qu'une microplaque, procédé dans lequel l'étape centrifugation est remplacée par l'application d'un champ magnétique, pouvant être entièrement automatisé et permettant de réaliser le phénotypage de globules rouges par lecture d'agglutination spécifique au moyen d'anticorps tests (sérum tests) agglutinants sans interférer significativement sur l'image finale obtenue, ce procédé pouvant être également applicable pour l'épreuve de Simonin (contre-épreuve) dans le cadre du phénotypage ABO. Un tel procédé serait d'autant plus avantageux si la composition magnétique utilisée ne provoque pas d'agrégation non spécifique des globules rouges en se liant avec eux après application du champ magnétique et, le cas échéant, évitant l'encombrement des sites antigéniques de ces globules rouges. Ce procédé serait d'autant encore plus avantageux si la composition magnétique utilisée permettait également en sa présence de réaliser une technique d'immunoadhérence de globules rouges sur phase solide, notamment pour la RAI et/ou pour l'épreuve de compatibilité, ou encore pour le phénotypage nécessitant l'utilisation de sérum tests non-agglutinants, ceci sans que cette composition magnétique interfère significativement sur l'image de la réaction finale obtenue avec ce type de technique.

Ceci est justement l'objet de la présente invention.

Les inventeurs ont mis en évidence que des érythrocytes mis en contact avec une solution diluée et aqueuse de ferrofluide exempt d'agent tensio-actif, obtenu à partir d'un mélange polyoxoanions de Fe(III) et d'au moins un métal M(II) de degré II d'oxydation (tel que Fe(II)), ferrofluide tel qu'obtenu notamment par les procédés décrits aux exemples 1 à 8 de la demande de brevet français publiée sous le No. 2 461 521 ou dans les exemples ci-après, ceci et contrairement au procédé décrit dans le document brevet EP 0 230 768, en l'absence d'addition de composé permettant de lier non spécifiquement les particules magnétiques du ferrofluide aux érythrocytes, étaient non seulement entraînés avec les particules de ferrofluide au fond du container les contenant sous l'action d'un champ magnétique induit par un aimant situé à l'extérieur et sous ledit container, mais que de plus cet entraînement provoquait simultanément l'agglutination spécifique de ces érythrocytes lorsque ces érythrocytes étaient également mis en contact avec un anticorps anti-antigène de groupe sanguin agglutinant, de type IgM, dirigé contre un antigène porté par ces érythrocytes.

Les inventeurs ont mis en évidence également et de manière inattendue que la présence des particules magnétiques contenues dans la solution ainsi diluée de ferrofluide, n'interférait pas de manière significative sur cette agglutination spécifique, cette dernière pouvant être mise en évidence aisément à l'oeil ou par tout système de lecture automatisé adéquate capable de détecter la présence ou l'absence d'agglutinats érythrocytaires.

Les inventeurs ont également mis en évidence de manière surprenante qu'une telle solution diluée de ferrofluide n'interférait pas non plus de manière significative sur la capacité des érythrocytes à se fixer sur une phase solide revêtue d'un ligand capable de reconnaître spécifiquement un composé fixé à la surface de ces érythrocytes lorsque ces derniers sont en présence des particules magnétiques contenues dans ce ferrofluide (technique d'immunoadhérence en phase solide).

Ainsi, la présente invention a pour objet un procédé de phénotypage de groupe sanguin, pouvant inclure l'épreuve dite de Simonin, caractérisé en ce qu'il comprend les étapes suivantes

a) Le mélange d'une suspension d'érythrocytes contenus dans l'échantillon avec une solution agueuse de ferrofluide, ledit ferrofluide étant obtenu ou susceptible d'être obtenu à partir d'un mélange polyoxoanions de Fe(III) et d'au moins un métal M(II) de degré II d'oxydation, de manière à obtenir une suspension homogène desdits érythrocytes dans la solution de ferrofluide,

b) la mise en contact dans un container de la suspension d'érythrocytes obtenue à l'étape a) avec un anticorps test anti-antigène de groupe sanguin connu. cette étape étant suivie d'une étape d'incubation ;

c) l'agitation de la suspension obtenue à l'étape b) après incubation ;

d) l'application d'un champ magnétique audit container de telle manière que les particules de ferrofluide soient entraînées au fond dudit container ;

e) l'agitation de la suspension obtenue à l'étape d) ;

f) la lecture à l'oeil et/ou par tout autre système de lecture approprié de la présence éventuelle d'agglutinats érythrocytaires dans le container.

Ledit ferrofluide est tel qu'obtenu notamment par les procédés décrits aux exemples 1 à 8 de la demande de brevet français publiée sous le No. 2 461 521 ou dans les exemples ci-après.

Dans la présente description les termes érythrocyte, globule rouge et hématie seront employés indifféremment pour désigner la même cellule sanguine.

De préférence la solution de ferrofluide qui sera utilisée dans les procédés de l'invention résultera de la dilution également en milieu aqueux et de préférence également en absence de tensio-actif (ou détergent) pour éviter notamment la lyse des érythrocytes lorsque ces derniers seront mis en contact avec cette solution de ferrofluide.

Par solution diluée en milieu aqueux sans agent tensio-actif de ferrofluide en solution aqueuse sans agent tensio-actif, on entend vouloir préciser ici que le ferrofluide, directement obtenu ou susceptible d'être directement obtenu par les procédés de préparation de ferrofluide décrits aux exemples 1 à 8 de la demande de brevet français publiée sous le No. 2 461 521 ou dans les exemples ci-après, est une solution aqueuse ne comprenant pas d'agent tensio-actif et que la solution utilisée après l'obtention de ce ferrofluide par lesdits procédés dans le but de diluer ce ferrofluide avant utilisation dans les procédés de la présente invention est une solution également aqueuse ne comprenant pas d'agent tensio-actif.

Dans un mode de réalisation préféré, ladite solution de ferrofluide sera caractérisée en ce qu'elle est préparée à partir d'un mélange polyoxoanions de Fe(III) et d'au moins un métal M(II) de degré II d'oxydation de préférence choisi parmi les métaux de la première série des métaux de transition tels que Fe(II), Co(II), Mn(II), Cu(II) ou Ni(II).

De préférence, ladite solution de ferrofluide est caractérisée en ce qu'elle est préparée à partir d'un mélange de polyoxoanions de Fe(III) et d'au moins un métal M(II) associé à un cation tel que $H^+$, $CH_3^+$, $N(CH_3)_4^+$, $N(C_2H_5)_4^+$ ou tout autre cation capable de conférer au polyoxoanion une plus grande solubilité dans l'eau que les cations Na, $K^+$ et $NH_4^+$, ces cations pourront être notamment apportés par les acides appropriés, tels que HCl, $CH_3COOH$, ou encore par l'hydroxyde de tétraméthyl- ou tétraéthyl-ammonium.

De préférence, ladite solution de ferrofluide est caractérisée en ce que les sources de métaux de départ choisis pour Fe(III) et M(II) pour préparer ledit ferrofluide seront des sels choisis parmi :

- pour Fe(III), ceux listés dans de la demande de brevet français publiée sous le No. 2 461 521, page 4, lignes 1 et 2, notamment le chlorure ferrique, et
- pour M(II), ceux listés dans de la demande de brevet français publiée sous le No. 2 461 521, page 4, lignes 3 à 6, notamment le chlorure ferreux.

De préférence, ladite solution de ferrofluide est préparée à partir d'un mélange entre Fe(III) et le métal M(II) caractérisé en ce que le rapport molaire initial de degré II est de $2 \pm 1$ ; de manière plus préférée de $2 \pm 0,5$ ; $2 \pm 0,25$ ou encore $2 \pm 0,1$, un rapport molaire initial de 2 entre Fe(III) et le métal M(II) de degré II étant le plus préféré.

Au mélange initial de sel de Fe(III) et du métal M(II) de degré II pourra être ajoutée une base forte appropriée telle que l'hydroxyde de sodium, l'hydroxyde de tétraméthyl- ou tétraéthyl-ammonium.

De préférence, le procédé selon l'invention est caractérisé en ce que ledit ferrofluide est obtenu par les procédés décrits aux exemples 1 à 8 de la demande de brevet français publiée sous le No. 2 461 521 ou par ceux tels que décrits ci-après dans les exemples.

De préférence encore, le procédé selon l'invention est caractérisé en ce que ladite solution aqueuse de ferrofluide est préalablement diluée dans un tampon ou une solution physiologique.

Dans certaines applications, notamment lorsqu'il s'agira d'améliorer la sensibilité ou la vitesse de réaction (sans toutefois augmenter la non spécificité du test), on pourra utiliser un tampon dit de basse force ionique (BFI), appelée encore tampon LISS (« LISS » pour « Low Ionic Strength Solution »).

Par tampon ou solution physiologique, l'homme de l'art comprendra qu'il s'agit là de tampon habituellement utilisé dans le domaine de la biologie cellulaire, notamment en immunohématologie afin d'éviter la lyse des globules rouges. De tels tampons ou solutions seront par exemple des tampons à pH physiologique, compris entre 6,8 et 7,5, avec une molarité ajustée de telle sorte qu'elle s'apparente à la molarité d'une solution de type NaCl à 9 pour mille (proche de 0,15 M de NaCl). On peut notamment citer mais sans s'y limiter le tampon phosphate type PBS à pH 7 - 7,4 bien connu de l'homme de l'art.

La composition des tampons BFI ou LISS ne sera pas ici développée, ces tampons étant bien connus en immunohématologie pour favoriser les réactions d'agglutination. Ces tampons sont notamment disponibles auprès des fournisseurs de réactifs pour l'immunohématologie (on pourra citer pour exemple, mais sans s'y limiter le tampon LISS de composition suivante : 16 g/l de glycine, 0,03 M de NaCl et 0,015 M phosphate à pH 6, 7).

Dans un mode de réalisation préféré, le procédé de l'invention est caractérisé en ce que ladite solution de ferrofluide est diluée de 0,25 à 10 % (v/v) dans ledit tampon ou ladite solution physiologique, de préférence entre 0,25 et 5 %, entre 0, 25 et 2,5 %, entre 0,25 et 1 %, entre 0,25 et 0,75 %.

Les dilutions comprises entre 0,25 % et 1 %, notamment 0,5 % sont particulièrement préférées pour le phénotypage par un procédé d'agglutination. On peut noter également que pour l'épreuve de compatibilité (détermination de la compatibilité directe entre le sérum du receveur et le globule rouge du donneur, voir ci-avant), des dilutions supérieures à 1 %, ou comprises entre 2 % et 10 % peuvent également être mises en oeuvre.

Pour le procédé selon l'invention pour la détermination par réaction d'agglutination d'érythrocytes de la présence d'un antigène de groupe sanguin ou d'un anticorps anti-antigène de groupe sanguin, on préfère également en ce que le container à réaction est une cupule de microplaque, de préférence une cupule à fond hémisphérique (« fond rond ») ou à fond en V.

Dans le procédé selon l'invention l'application du champ magnétique sera de préférence obtenue par un aimant, de type permanent, situé à l'extérieur et en-dessous du ou des containers à réaction, ledit aimant étant de préférence encore de magnitude comprise entre 8 000 et 16 000 gauss, de préférence entre 10 000 et 14 000 gauss, 12 000 gauss étant la magnitude préférée.

Par procédé de détermination par réaction d'agglutination d'érythrocytes de la présence d'un antigène de groupe sanguin à la surface d'un érythrocyte d'un échantillon , on entend désigner ici de façon particulière un procédé de phénotypage érythrocytaire (ou encore dénommée ici phénotypage sanguin ou de globules rouges), qui correspond à la recherche et à l'identification des antigènes de groupe sanguin à la surface des hématies (à l'exception du système particulier ABO où l'on recherche en outre la présence des anticorps réguliers correspondants (épreuve dite de Simonin)), ceci concernant aussi bien le receveur que le donneur.

Dans la présente description, on entend désigner par un anticorps test anti-antigène de groupe sanguin connu, des anticorps polyclonaux, monoclonaux ou encore recombinants de spécificité connue capable de reconnaître et se fixer sur l'érythrocyte portant l'antigène contre lequel il est dirigé.

Ces anticorps tests anti-antigènes de groupe sanguin connu pourront se présenter sous une forme liquide en solution ou sous forme desséchée dans le container servant de support pour la réaction d'agglutination ou, le cas échéant, dans le support servant à la mise en contact des érythrocytes à phénotyper avec ledit anticorps.

De préférence encore ces anticorps seront de type agglutinant, tels que des IgM, dans les procédés de l'invention par réaction d'agglutination, c'est-à-dire capables d'agglutiner une suspension d'érythrocytes portant l'antigène de groupe contre lequel la spécificité dudit anticorps est dirigée, ceci sans l'aide d'une anti-immunoglobuline et dans les conditions appropriées bien connues de l'homme de l'art ou indiquées sur la fiche technique du fournisseur attachée à l'utilisation d'un tel anticorps (tel que par exemple en milieu salin à température ambiante ou encore à 37°C, etc.).

Dans les procédés de phénotypage selon l'invention où il sera préféré ou nécessaire de réaliser pour certains antigènes de groupe sanguin le phénotypage selon l'invention par une technique de type immunoadhérence sur phase solide, notamment :

- lorsque ledit phénotypage sera réalisé à l'aide d'un container pour la réaction finale dont la paroi interne inférieure sera préalablement revêtue d'une anti-immunoglobuline ou de tout ligand capable de reconnaître spécifiquement et de fixer le complexe spécifique anticorps-test/érythrocyte ; ou
- lorsque ledit phénotypage sera réalisé à l'aide d'un container pour la réaction finale dont la paroi interne inférieure sera préalablement revêtue dudit anticorps-test capable de reconnaître spécifiquement et de fixer l'érythrocyte portant l'antigène correspondant à l'anticorps-test,

lesdits anticorps tests anti-antigène de groupe sanguin connu pourront être de nature non agglutinante, tels que des IgG ou l'un de leurs fragments capable de reconnaître ledit antigène.

Dans un procédé particulier, le mélange à l'étape a) dudit procédé pourra être réalisé préalablement au test, 1 heure, voire plusieurs heures, un jour ou plusieurs jours à l'avance, ou, le cas échéant, pourrra être obtenu auprès d'un fournisseur, ceci lorsqu'il s'agira de mélanger une suspension d'érythrocytes tests de groupe connu, avec une solution de ferrofluide. Il en sera de même pour tous les procédés.

Dans ce procédé pour la détermination par réaction d'agglutination d'érythrocytes de la présence d'un antigène de groupe sanguin selon l'invention, ladite solution de ferrofluide utilisée à l'étape a) est une solution diluée de ferrofluide directement obtenu ou susceptible d'être directement obtenu par les procédés de préparation de ferrofluide décrits aux exemples 1 à 8 de la demande de brevet français publiée sous le N° 2 461 521 ou dans les exemples ci-après.

Dans ce procédé pour la détermination par réaction d'agglutination d'érythrocytes de la présence d'un antigène de groupe sanguin selon l'invention, on préfère en ce qu'avant l'étape b), ladite suspension d'érythrocytes contenus dans l'échantillon est préalablement soumise à l'action d'une enzyme protéasique.

Dans ce procédé pour la détermination par réaction d'agglutination d'érythrocytes de la présence d'un antigène de groupe sanguin selon l'invention, on préfère également en ce que lorsque la suspension obtenue à l'étape a) est une suspension d'érythrocytes issus de l'échantillon, ladite suspension d'érythrocytes est soumise à l'action de l'enzyme protéasique à la fin de l'étape a).

Dans ce procédé pour la détermination par réaction d'agglutination d'érythrocytes de la présence d'un antigène de groupe sanguin selon l'invention, on préfère également en ce qu'à l'étape b), les anticorps tests anti-antigène de groupe sanguin sont contenus sous forme desséchée dans le container avant à la mise en contact de la suspension obtenue à l'étape a) si cette suspension est une suspension d'érythrocytes issus de l'échantillon.

Dans ce procédé pour la détermination par réaction d'agglutination d'érythrocytes de la présence d'un antigène de groupe sanguin selon l'invention, on préfère également en ce que les anticorps tests anti-antigène de groupe sanguin connu sont des anticorps agglutinants, de préférence de type IgM.

Dans ce procédé pour la détermination par réaction d'agglutination d'érythrocytes de la présence d'un antigène de groupe sanguin selon l'invention, on préfère également en ce qu'à l'étape d'incubation à la fin de l'étape b) est effectuée pendant un temps compris entre 5 et 15 min., de préférence 10 min. à température ambiante.

Dans ce procédé pour la détermination par réaction d'agglutination d'érythrocytes de la présence d'un antigène de groupe sanguin selon l'invention, on préfère également en ce que l'étape c) d'agitation est effectuée pendant un temps compris entre 30 sec. et 2 min. 30 sec., de préférence pendant 1 min. à 2 min..

Dans ce procédé pour la détermination par réaction d'agglutination d'érythrocytes de la présence d'un antigène de groupe sanguin selon l'invention, on préfère également en ce que l'étape d) d'application d'un champ magnétique audit container est réalisée au moyen d'un aimant situé à l'extérieur sous le container de telle manière que les particules de ferrofluide soient entraînées au fond dudit container selon un axe vertical.

Dans ce procédé pour la détermination par réaction d'agglutination d'érythrocytes de la présence d'un antigène de groupe sanguin selon l'invention, on préfère également en ce qu'à l'étape d) ledit aimant est un aimant permanent de magnitude comprise entre 10 000 et 14 000 gauss, de préférence 12 000 gauss.

Dans ce procédé pour la détermination par réaction d'agglutination d'érythrocytes de la présence d'un antigène de groupe sanguin selon l'invention, on préfère également en ce qu'à l'étape d) le champ magnétique est appliqué pendant 2,5 min. à 10 min., de préférence pendant 4 min. à 6 min. audit container.

Dans ce procédé pour la détermination par réaction d'agglutination d'érythrocytes de la présence d'un antigène de groupe sanguin selon l'invention, on préfère également en ce qu'à l'étape e), l'agitation de la suspension avant lecture est effectuée en deux étapes d'agitation successives, la deuxième étant moins longue et moins vigoureuse que la première.

Dans ce procédé pour la détermination par réaction d'agglutination d'érythrocytes de la présence d'un antigène de groupe sanguin selon l'invention, on préfère également en ce qu'à l'étape a), ladite suspension d'érythrocytes issus d'érythrocytes contenus dans l'échantillon est issue d'un culot d'érythrocytes obtenu après sédimentation ou centrifugation, de préférence par sédimentation, d'un échantillon de sang total d'un individu.

Dans ce procédé pour la détermination par réaction d'agglutination d'érythrocytes de la présence d'un antigène de groupe sanguin selon l'invention, on préfère également en ce qu'à l'étape b), ledit container est une cupule de microplaque, de préférence une cupule à fond hémisphérique (« fond rond »).

Dans ce procédé pour la détermination par réaction d'agglutination d'érythrocytes de la présence d'un antigène de groupe sanguin selon l'invention, on préfère également en ce qu'à l'étape c), l'agitation est effectuée à l'aide d'un agitateur pour microplaque à une vitesse comprise entre 500 et 800 trs/min., de préférence entre 650 et 750 trs/min..

Dans ce procédé pour la détermination par réaction d'agglutination d'érythrocytes de la présence d'un antigène de groupe sanguin selon l'invention, on préfère également en ce qu'à l'étape e), l'agitation est effectuée en 2 étapes à l'aide d'un agitateur pour microplaque, une première d'agitation pendant 1 à 2 min. 30 sec. à une vitesse comprise entre 800 et 1000 trs/min., de préférence 1 min. 45 sec. à une vitesse de 900 trs/min., et une deuxième agitation pendant 30 sec. à 1 min. à une vitesse comprise entre 350 et 550 trs/min., de préférence 45 sec. à une vitesse de 450 trs/min..

Dans ce procédé pour la détermination par réaction d'agglutination d'érythrocytes de la présence d'un antigène de groupe sanguin selon l'invention, on préfère également en ce qu'à l'étape b) :

- on met en contact dans ladite cupule de 40 µl à 50 µl de la suspension d'érythrocytes obtenue à l'étape a) avec des anticorps tests anti-antigène de groupe sanguin connu contenus sous forme desséchée au fond de la cupule, si la suspension d'érythrocytes obtenue à l'étape a) est une suspension d'érythrocytes issus de l'échantillon ; et, le cas échéant,

- on met en contact dans ladite cupule de 20 µl à 30 µl de la suspension d'érythrocytes obtenue à l'étape a) avec 20 µl à 30 µl de plasma ou de sérum de l'échantillon susceptible de contenir les anticorps anti-antigène de groupe sanguin, si la suspension d'érythrocytes obtenue à l'étape a) est une suspension d'érythrocytes tests de groupe sanguin connu.

Sous un mode particulier de réalisation, la présente invention a pour objet un procédé selon l'invention pour le groupage sanguin ABO d'un échantillon de sang total issu d'un individu, caractérisé en ce que à l'étape b) :

- on met en contact dans autant de containers distincts la suspension d'érythrocytes issus de l'échantillon obtenue à l'étape a) avec un anticorps test anti-A et un anticorps test anti-B ; et

- on met en contact dans autant de containers distincts un échantillon de plasma ou de sérum de l'individu avec une suspension d'érythrocytes test de groupe A, de préférence A1, une suspension d'érythrocytes test de groupe B, et, le cas échéant, une suspension d'érythrocytes test de groupe O,

caractérisé en ce que à l'étape f) on évalue par lecture à l'oeil et/ou par tout autre système de lecture approprié la présence éventuelle d'agglutinats érythrocytaires dans chacun desdits containers, l'ensemble de ces lectures permettant de déterminer le groupe ABO de l'individu.

Sous un mode également particulier de réalisation, la présente invention a pour objet un procédé selon l'invention pour le phénotypage sanguin, hors épreuve de Simonin, d'un échantillon de sang total issu d'un individu, caractérisé en ce que à l'étape b) :

- on met en contact dans autant de containers distincts la suspension d'érythrocytes issus de l'échantillon obtenue à l'étape a) avec un anticorps test spécifique de l'antigène de groupe sanguin dont on cherche à déterminer la présence ou l'absence sur les érythrocytes de l'échantillon, chaque container contenant une seule spécificité d'anticorps,

caractérisé en ce que à l'étape f) on évalue par lecture à l'oeil et/ou par tout autre système de lecture approprié la présence éventuelle d'agglutinats érythrocytaires dans chacun desdits containers, l'ensemble de ces lectures permettant de déterminer le phénotype de l'individu pour les antigènes de groupe sanguin recherchés.

Sous un mode également particulier de réalisation, la présente invention a pour objet un procédé selon l'invention pour le phénotypage sanguin, épreuve de Simonin comprise, d'un échantillon de sang total issu d'un individu, caractérisé en ce que à l'étape b) :

- on met en contact dans autant de containers distincts la suspension d'érythrocytes issus de l'échantillon obtenue à l'étape a) avec un anticorps test anti-A et un anticorps test anti-B, et
- on met en contact dans autant autres containers distincts la suspension d'érythrocytes issus de l'échantillon obtenue à l'étape a) avec un anticorps test spécifique de l'antigène de groupe sanguin, hors antigène A et B, dont on cherche à déterminer la présence ou l'absence sur les érythrocytes de l'échantillon, chaque autre container contenant une seule spécificité d'anticorps ; et
- on met en contact dans autant de containers distincts un échantillon de plasma ou de sérum de l'individu avec une suspension d'érythrocytes test de groupe A, une suspension d'érythrocytes test de groupe B, et, le cas échéant, une suspension d'érythrocytes test de groupe O,

caractérisé en ce que à l'étape f) on évalue par lecture à l'oeil et/ou par tout autre système de lecture approprié la présence éventuelle d'agglutinats érythrocytaires dans chacun desdits containers, l'ensemble de ces lectures permettant de déterminer le groupe ABO de l'individu et de déterminer le phénotype de l'individu pour les autres antigènes de groupe sanguin recherchés.

Il est aussi décrit un procédé pour la recherche et/ou l'identification d'anticorps irrégulier dans un échantillon de plasma ou de sérum d'un receveur, caractérisé en ce qu'il comprend les étapes suivantes :

a) l'incubation dans un premier container dudit échantillon de plasma ou de sérum avec une suspension d'érythrocytes tests de phénotype connu dans des conditions permettant aux anticorps irrégulier susceptibles d'être présents de se fixer spécifiquement sur les érythrocytes tests ;

b) l'ajout d'une solution de ferrofluide au mélange obtenu à l'étape a) de manière à obtenir une suspension homogène desdits érythrocytes dans la solution de ferrofluide ;

c) l'application d'un champ magnétique audit container au moyen d'un aimant situé à l'extérieur et sous ledit container de manière à entraîner lesdits érythrocytes avec les particules magnétiques contenues dans le ferrofluide au fond dudit container (plaquage des érythocytes), suivie, le cas échéant d'une élimination du surnageant obtenu ;

d) le cas échéant, le lavage du culot d'érythrocytes et de particules magnétiques par ajout d'une solution lavage de, suivi de l'élimination du surnageant après plaquage des érythrocytes, cette étape pouvant être renouvelée plusieurs fois ;

e) le transfert dans un deuxième container d'un échantillon ou de la totalité de la suspension d'érythrocytes et de ferrofluide obtenue à l'étape c) ou d), lequel deuxième container étant revêtu sur sa paroi interne inférieure d'une anti-globuline capable de reconnaître et de fixer sur cette paroi les complexes anticorps irrégulier/érythrocytes tests éventuellement présents ;

f) l'application d'un champ magnétique audit deuxième container de telle manière que les érythrocytes soient entraînés au fond dudit container avec les particules magnétiques contenues dans le ferrofluide; et

g) la lecture à l'oeil et/ou par tout autre système de lecture approprié de la répartition des érythrocytes la paroi inférieure du container.

Dans ce procédé, la présence d'un seul point rouge situé dans l'axe vertical du container et en son fond pourra être interprétée comme une abscence d'anticorps iirégulier ;

En revanche, la présence significative d'érythrocytes répartis sur toute la partie du container revêtu d'anti-immunoglobuline (ou d'anti-espèce, voir la variante ci-après) pourra être interprétée comme une présence d'anticorps irréguliers.

Il est aussi décrit à un procédé pour la recherche et/ou l'identification d'anticorps irréguliers selon la présente invention, caractérisé en ce que l'étape b) d'adjonction d'une solution de ferrofluide à la suspension desdits érythrocytes tests est effectuée préalablement avant l'ajout dudit échantillon de sérum ou de plasma.

Un procédé pour la recherche et/ou l'identification d'anticorps irréguliers peut comprendre les étapes suivantes:

- avant l'étape e), on met en contact dans le premier container la suspension d'érythrocytes lavée obtenue à l'étape précédente avec une solution d'anti-immunoglobuline, et, le cas échéant, après agitation et incubation ;
- on transfère dans le deuxième container un échantillon ou la totalité de la suspension d'érythrocytes et de ferrofluide obtenue, lequel deuxième container étant revêtu sur sa paroi interne inférieure d'une anti-globuline, de préférence un anticorps anti-espèce, capable de reconnaître et de fixer sur cette paroi les complexes anti-immunoglobuline/anticorps irrégulier/érythrocytes tests éventuellement présents.

**EP 1 745 290 B1**

Il est également décrit un procédé pour déterminer la compatibilité entre un concentré érythrocytaire d'un donneur et un receveur, caractérisé en ce qu'il comprend les étapes du procédé pour la recherche et/ou l'identification d'anticorps irréguliers telles que décrit précédemment mais dans lequel à l'étape a),

on incube dans ledit premier container un échantillon de plasma ou de sérum du receveur avec une suspension d'érythrocytes du donneur dans des conditions permettant aux anticorps susceptibles d'être présents et dirigés contre un antigène de groupe sanguin présent sur les érythrocytes du donneur de se fixer sur lesdits érythrocytes, les étapes suivantes et leurs variantes étant identiques au procédé pour la RAI décrit précédemment.

Sous un autre aspect la présente invention concerne un dispositif pour la détermination par réaction d'agglutination d'érythrocytes de la présence d'un antigène du groupe sanguin à la surface d'un érythrocyte d'un échantillon caractérisé en ce qu'il comprend :

a) un container contenant une solution de ferrofluide ;
b) un container contenant une suspension d'érythrocytes de l'échantillon à tester ;
c) - un ou plusieurs containers à réaction contenant un anticorps test anti-antigène de groupe sanguin. Cet anticorps peut être, sous forme desséchée.
Le dispositif peut contenir un ou plusieurs containers contenant une suspension d'érythrocytes test de groupe connu ;
d) au moins un aimant ou un ensemble d'aimants pouvant être disposé à l'extérieur dudit ou desdits containers et sous ledit ou lesdits containers ;
e) un système d'agitation dudit ou desdits containers.

Le dispositif peut aussi comprendre un lecteur capable d'évaluer la présence d'agglutinats érythrocytaires dans chacun des containers.

De préférence, le dispositif selon l'invention est caractérisé en ce que ledit container à réaction est une cupule de microplaque, cette dernière pouvant être à fond hémisphérique.

Sous un mode particulier de réalisation, l'invention a pour objet un dispositif pour le groupage sanguin ABO d'un échantillon de sang total issu d'un individu, caractérisé en ce que :

- lesdits containers contenant une suspension d'érythrocytes tests de groupe connu contiennent distinctement une suspension d'érythrocytes tests de groupe A, une suspension d'érythrocytes tests de groupe B. Lesdits containers peuvent contenir une suspension d'érythrocytes test de groupe O.
- lesdits containers contenant un anticorps test anti-antigène de groupe sanguin contiennent distinctement un anticorps anti-A et un anticorps anti-B.

Sous un mode également particulier de réalisation, l'invention a pour objet un dispositif pour le phénotypage sanguin, hors épreuve de Simonin, d'un échantillon de sang total issu d'un individu, caractérisé en ce que :

- lesdits containers contiennent distinctement un anticorps test spécifique de l'antigène de groupe sanguin dont on cherche à déterminer la présence ou l'absence sur les érythrocytes de l'échantillon.

Sous un mode encore particulier de réalisation, l'invention a pour objet un dispositif pour le phénotypage sanguin, épreuve de Simonin comprise, d'un échantillon de sang total issu d'un individu, caractérisé en ce que :

- lesdits containers contenant une suspension d'érythrocytes tests de groupe connu contiennent distinctement une suspension d'érythrocytes tests de groupe A, une suspension d'érythrocytes tests de groupe B. Une suspension d'érythrocytes test de groupe O peut être ajoutée;
- lesdits containers contenant un anticorps test anti-antigène de groupe sanguin contiennent distinctement un anticorps anti-A, un anticorps anti-B et un anticorps test spécifique de l'antigène de groupe sanguin, hors ABO, dont on cherche à déterminer la présence ou l'absence sur les érythrocytes de l'échantillon.

Sous un autre aspect, la présente invention a pour objet un kit ou nécessaire à réactifs pour la détermination par réaction d'agglutination d'érythrocytes de la présence d'un antigène du groupe sanguin à la surface d'un érythrocyte d'un échantillon caractérisé en ce qu'il comprend :

a) un ou plusieurs containers à réaction contenant distinctement un anticorps test anti-antigène de groupe sanguin ;
b) une solution de ferrofluide. Le ferrofluide peut être dilué dans un tampon ou une solution physiologique, par exemple dans un tampon basse force ionique ;
Une suspension d'érythrocytes tests de groupe sanguin connu peut être ajoutée :
c) un ou plusieurs aimants pouvant être disposés à l'extérieur sous le ou les containers à réaction.

L'invention concerne particulièrement un kit ou nécessaire à réactifs selon l'invention, pour le groupage sanguin ABO d'un échantillon de sang total issu d'un individu, caractérisé en ce que :

- lesdits containers contenant une suspension d'érythrocytes tests de groupe connu contiennent distinctement une suspension d'érythrocytes tests de groupe A une suspension d'érythrocytes tests de groupe B Une suspension d'érythrocytes test de groupe O peut être ajoutée.
- lesdits containers contenant un anticorps test anti-antigène de groupe sanguin contiennent distinctement un anticorps anti-A et un anticorps anti-B.

L'invention concerne aussi un kit ou nécessaire à réactifs selon l'invention, pour le phénotypage sanguin, épreuve de Simonin comprise, d'un échantillon de sang total issu d'un individu, caractérisé en ce que il comprend en outre au moins autant de containers contenant distinctement anticorps test spécifique de l'antigène de groupe sanguin, autre que l'antigène A et B, dont on cherche à déterminer la présence ou l'absence sur les érythrocytes de l'échantillon à phénotyper.

De préférence, le kit ou nécessaire à réactifs selon l'invention est caractérisé en ce que lesdits anticorps test anti-antigène de groupe sanguin contenus dans lesdits containers sont sous forme desséchée.

De préférence encore, le kit ou nécessaire à réactifs selon l'invention, est caractérisé en ce que lesdits containers sont des cupules de microplaque. Ces cupules peuvent être à fond hémisphérique.

Les exemples qui suivent sont destinés à illustrer l'invention sans aucunement en limiter la portée.

**EXEMPLE 1** : Fabrication du ferrofluide prépurifié

Matériel et méthode

Fabrication du Ferrofuide :

Matériel particulier et produits

- 1 filtre Stericup GS 200 ml 0,22 $\mu$m (Réf. : 107943)
- 1 aimant
- solution de $NH_4OH$ à 28-30 % (ACROS, Réf. : 205840025 lot n° A 017559001)
- solution de $HNO_3$ à 60 % (NORMAPUR, Réf. : UN 2031 lot n° N182)
- $FeCl_2$, $4H_2O$ (SIGMA, Réf. : 22,029-9 lot n° S18641-463)
- $FeCl_3$, $6H_2O$ (SIGMA, Réf. : F-2877, lot n° 033K0108)

Fabrication du Ferrofuide :

1/ Peser 13,51 g de $FeCl_3$ et dissoudre dans 20 ml d'eau déminéralisée et filtrée par agitation magnétique. Transférer la solution dans une éprouvette de 50 ml, rincer et compléter à 50 ml avec de l'eau déminéralisée filtrée (solution à 1 M).

2/ Peser 19,88 g de $FeCl_2$ et dissoudre dans 20 ml d'eau déminéralisée et filtrée par agitation magnétique. Transférer la solution dans une éprouvette de 50 ml, rincer et compléter à 50 ml avec de l'eau déminéralisée filtrée (solution à 2 M).

3/ Filtrer chaque solution sur filtre seringue 0,22 $\mu$m.

4/ Mettre 30 ml d'une solution d'ammoniac ($NH_4OH$) à 28 % dans une éprouvette de 250 ml et ajouter qsp 120 ml d'eau déminéralisée et filtrée (solution à 2 M).

5/ Peser le ballon en verre de 1 1 du Rotavapor à vide.

Incubation :

6/ Mettre 10 ml de la solution de $FeCl_2$ (2 M) et 40 ml de la solution de $FeCl_3$ (1 M) dans le ballon 1 L pour Rotavapor, homogénéiser la solution en agitant le ballon à la main.

7/ Ajouter 200 ml d'eau déminéralisée filtrée, homogénéiser la solution en agitant le ballon à la main.

8/ Placer le ballon de façon inclinée sur un appareil de type Rotavapor.

9/ Faire tourner le ballon à vitesse maximum pour bien homogénéiser la solution.

10/ Ajouter 120 ml de la solution d'ammoniac 2 M.

11/ Laisser tourner le ballon pendant 15 minutes.

12/ Mettre 105 ml d'acide nitrique (HNO$_3$) à 60 % dans une fiole de 1 1 et ajouter qsp 11 d'eau déminéralisée filtrée (solution à 1 M).

13/ Après les 15 minutes de rotation, placer le ballon sur l'aimant pendant 4 minutes.

14/ Aspirer la totalité du surnageant.

15/ Resuspendre le culot.

1er lavage :

16/ Placer le ballon sur le Rotavapor et le faire tourner à vitesse maximum.

17/ Ajouter 200 ml de la solution d'HNO$_3$ à 1 M.

18/ Laisser tourner le ballon pendant 10 minutes.

19/ Resuspendre le culot.

20/ Répéter l'opération jusqu'à dissolution complète du culot.

21/ Placer le ballon sur pour faire décanter la suspension de fer.

22/ Aspirer lesurnageant.

23/ Resuspendre le culot.

Les étapes 16 à 23 peuvent être répétées si nécessaire.

Dilution finale :

26/ Peser le ballon.

27/ En déduire la masse du culot obtenu et calculer le rendement de la réaction :

$$\text{(Masse culot obtenu / Masse de fer initiale)} \times 100$$

$$\text{Masse de fer initiale} = 14{,}77 \text{ g}$$

$$55\% < \text{rendement}$$

28/ Ajouter 200 ml d'eau filtrée dans le ballon.

29/ Placer le ballon sur le Rotavapor.

30/ Resuspendre le culot.

31/ filtrer la solution sur un filtre Stericup à 0,22 $\mu$m.

Préparation de la solution mère de ferrofluide :

Diluer le ferrofluide ainsi obtenu dans un tampon LISS ou une solution physiologique à la concentration voulue dans un flacon. Noter sur une étiquette collée sur le flacon la concentration de ferrofluide, le numéro de lot de ferrofluide et la date de fabrication.

Conservation du ferrofluide et/ou de la solution diluée à 4°C.

**EXEMPLE 2 :** Phénotypage de 288 échantillons : 144 donneurs + 144 receveurs par Technique Automatisée (sur automate de typeTECAN)

- Automate TECAN équipé d'une plaque de 96 aimants isopôles alternés en Nord-Sud (12 000 Gauss), d'un Téléshake Variomag H+P Lab et d'un lecteur de microplaque.
- Prélèvements sanguins sur anticoagulant type EDTA
- mise en contact extemporanée des globules rouges (GR) de patients avec différents lots de ferrofluide dilué entre 0,3 et 0,5 % (v/v) dans du LISS ou dans un tampon physiologique.
- Contre-Epreuve : Hématies A1 et B d'un panel mise en contact extemporanée avec différents lots de ferrofluide dilué entre 0,3 et 0,5 % (v/v) dans du LISS ou dans un tampon physiologique, et conservées à 4°C à 1 % de GR (v/v).
- Aimantation de 5 minutes.
- Agitations sur Téléshake :

Avant aimantation : 1 min 30 sec, à 700 trs/min nwse.

Après aimantation 2 min à 900 trs/min nwse + 45 sec à 450 trs/min nwse.

Résultats :

La performance du test a été évaluée en comparaison avec une technique de référence choisie : dénommée DUO MICRO en technique manuelle.

Utilisation de 4 lots de ferrofluide dilués entre 0,34 et 0,45 % (v/v) dans du LISS ou dans un tampon physiologique préparés comme ci-avant, ceci de façon aléatoire sur les 288 échantillons.

Concordance avec la technique de référence supérieure ou égale à 98 % (6 rejets).
21 Kell positifs attendus $\Rightarrow$ 20 Kell positifs trouvés.

Aucun point de sédimentation.

Tableau comparatif des deux techniques :

| | | Technique avec ferrofluide | | | |
|---|---|---|---|---|---|
| | | + | - | Douteux | |
| Duo-Micro | + | 282 | 0 | 0 | 282 |
| | - | 0 | 0 | 4 (TN ; D) | 4 |
| | Douteux | 0 | 1 (K en DP) | 1 (C en DP) | 2 |
| | | 282 | 1 | 5 | **288** |

Identification des rejets observés :

- Un échantillon avec le Rh1 Douteux (valeur lecteur = 33) et son TN granuleux (valeur lecteur = 3).
- Un échantillon avec le Rh1 douteux (valeur lecteur = 28).
- Un échantillon avec une Double Population (DP) faible en C, non détectée sur automate (valeur lecteur = 6).
- Un échantillon avec le Rh1 granuleux (valeur lecteur = 15).
- Un échantillon avec une Double Population faible en Kell et non détectée sur automate.
- Un échantillon avec le TN qui granule (valeurs lecteur =17).

Il est à noter que les tubes avec les doubles populations, contenaient très peu de culot globulaire, ce qui peut induire des problèmes de prélèvement dans le tube par les aiguilles du TECAN.

De plus les doubles populations n'ont été que très faiblement détectées par la technique de référence (Duo Micropar centrifugation) l'oeil et le lecteur les interprètent comme des Douteux.

Les granulations ne sont que très faiblement visibles à l'oeil et si nous rehaussons le seuil de négativité elles ne seront plus détectées par le lecteur.

**EXEMPLE 3** : Phénotypage d'échantillons : Technique Automatisée (sur automate de typeTECAN)

Matériel :

- Prélèvements de donneurs et receveurs sur tubes EDTA (acide Ethylène Diamine Tétraacétique)

- solutions de ferrofluides purs lavés

  - mise en contact extemporanée des globules rouges (GR) de patients avec différents lots de ferrofluide dilués entre 0,3 et 0,5 % (v/v) dans du LISS

- Hématies de Contre-Epreuve mises en contact extemporanées et conservées à 1 %, de préférence dans un tampon physiologique, à 4°C.
- Broméline Diagast code n° 69024.
- Microplaques Duo Micro lot 302 000.
- LISS Diagast code V6901B-1.
- Plaque de 96 puits (Deep Well, 700 µl) Greiner.

Protocole :

- Centrifuger les tubes de patients à 2 500 g pendant 5 min. à température ambiante
- Placer les tubes échantillons dans les portoirs
- Placer les tubes d'Hématies tests (Al et B) en suspension dans le ferrofluide pour la Contre-Epreuve sur un portoir
- Faire une solution magnétisante de ferrofluide avec une DO à 450 nm équivalente à 0,9 dans du LISS, à répartir dans 8 tubes à hémolyse en position n° 1 à 8 Grid n° 4.
- Remplir le Rack n° 2 de Broméline.
- Placer la plaque Deep Well sur le site n° 1 Grid n° 7 et les microplaques Duo Micro sur le site n° 2 et 3 Grid n° 7.
- Faire débuter le protocole Duo micro par le TECAN :

  - dépôt de 25 µl de plasma dans les puits de Contre Epreuve,
  - distribution, dans la Deep Well, de 240 µl de solution magnétisante par échantillon à tester,
  - ajout 10 µl de culot globulaire à la solution magnétisante,
  - homogénéisation de la suspension globulaire par plusieurs aspirations-refoulements,
  - ajout de 700 µl de broméline,
  - dépôt de 40 µl de suspension globulaire magnétisée et brominée dans les puits des 2 microplaques Duo Micro,
  - dépôt de 25 µl d'Hématies test dans les puits de la Contre-Epreuve,
  - incubation des microplaques 10 min.,
  - agitation de la microplaque sur le Teleshake à 700 trs/min pendant 1 min 30 sec.,
  - aimantation des microplaques pendant 5 min.,
  - agitation de la microplaque sur Teleshake à 900 trs/min pendant 2 min, puis à 450 trs/min pendant 45 sec..

- Lecture de la plaque sur lecteur, puis lecture à l'oeil.

**EXEMPLE 4 :** Phénotypage de 312 échantillons en Technique Manuelle

Protocole :

Fabrication du ferrofluide : cf. exemple 1

Faisabilité manuelle réalisée sur 312 échantillons : 160 donneurs + 152 receveurs

- Prélèvements sanguins sur EDTA
- Magnétisation extemporanée des GR de patients avec différents lots de solution mère de ferrofluide diluées dans du LISS de façon à avoir une dilution finale du ferrofluide entre 0,3 % et 0, 5 %
- Contre-Epreuve : Hématies A1 et B d'un panel mises en suspension dans un flacon en verre dans la solution de ferrofluide diluée (solution mère diluée à 0,3 % dans du Liss ou dans un tampon physiologique) et conservées à 4°C à 1 % de GR (dans du Liss, de préférence dans un tampon physiologique,
- Aimantation de 5 minutes
- Agitations sur Téléshake :

    avant aimantation : 1 min 30 sec à 700 trs/min nwse
    après aimantation : 2 min à 900 trs/min nwse + 45 sec à 450 trs/min nwse

Résultats :

La performance du test a été évaluée en comparaison avec la technique de référence choisie : DUO MICRO en technique manuelle.

Utilisation des 4 lots de ferrofluide cités ci-dessus de façon aléatoire sur les 312 échantillons.

Concordance avec la technique de référence = 98 %.

Rejet = 2 % (7 échantillons rejetés/312).

35 Kell positifs attendus $\Rightarrow$ 34 Kell positifs trouvés.

% de points de sédimentation = 0,4 % (15 puits/3744).

Tableau comparatif des 2 techniques :

| | | Technique « ferrofluide » | | | |
|---|---|---|---|---|---|
| | | + | - | Douteux (Dtx) | |
| Duo-Micro | + | 305 | 0 | 0 | 305 |
| | - | 0 | 0 | 5 (TN = granulations) | 5 |
| | Dtx | 0 | 1 (Kell en DP) | 1 (C en DP) | 2 |
| | | 305 | 1 | 6 | 312 |

Identification des rejets observés :

- Un échantillon avec une Double Population (DP) faible en C, interprété comme Douteux (valeur lecteur = 33) mais aussi interprété Douteux à l'oeil et au lecteur dans la technique de référence (valeur lecteur = 27).
- Un échantillon avec une Double population en Kell et non détecté (FN). Il est à noter que l'échantillon présentait très peu de volume dans le tube, ce qui rend le prélèvement du culot très difficile et peut entraîner une diminution de l'hématocrite à cette étape du protocole.
- 5 échantillons ont présenté des granulations dans les TN (valeurs lecteur = 18, 14, 12, 36 et 18) ainsi que dans toutes les spécificités pour lesquelles ils étaient négatifs (les valeurs lecteur étaient < 20). Deux de ces échantillons étaient sur la même plaque.

CONCLUSION :

Au terme de cette étude, nous pouvons voir que les performances de la technique utilisant le ferrofluide pour le phénotypage sont très proches de celles de la technique de référence utilisée en comparaison.

Si la technique « ferrofluide » n'a pas détectée certaines doubles populations très faibles, alors que la technique de référence les détectait douteux, on peut noter néanmoins que ces 2 faux négatifs peuvent s'expliquer par le fait que les tubes échantillons étaient très peu remplis et le volume de culot globulaire étant faible, l'hématocrite de l'échantillon devait être trop faible.

En général, et sur l'ensemble de la population de receveurs testés, 30 % sont des échantillons présentant des doubles populations qui sont toujours détectées en technique « ferrofluide ».

En ce qui concerne les échantillons rejetés suite à des granulations, celles-ci sont faiblement visibles à l'oeil et le lecteur les rejette car elles dépassent le seuil de négativité fixé à 10. Actuellement ce seuil de négativité est fixé à 15 voire à 20 sur tous les lecteurs. Dans le cas où nous fixerions le seuil à ces mêmes valeurs le taux de rejet serait inférieur à 1 %.

**Revendications**

1. Procédé de phénotypage de groupe sanguin dans lequel procédé, on détermine par réaction d'agglutination d'érythrocytes la présence d'un antigène de groupe sanguin à la surface d'un érythrocyte d'un échantillon, **caractérisé en ce qu'**il comprend les étapes suivantes :

   a) le mélange d'une suspension d'érythrocytes contenus dans l'échantillon avec une solution aqueuse de ferrofluide obtenu ou susceptible d'être obtenu à partir d'un mélange polyoxoanions de Fe(III) et d'au moins un métal M(II) de degré II d'oxydation, de manière à obtenir une suspension homogène desdits érythrocytes dans la solution de ferrofluide :
   b) la mise en contact dans un container de la suspension d'érythrocytes obtenue à l'étape a) avec un anticorps test anti-antigène de groupe sanguin connu, cette étape étant suivie d'une étape d'incubation ;
   c) l'agitation de la suspension obtenue à l'étape b) après incubation ;
   d) l'application d'un champ magnétique audit container de telle manière que les particules magnétiques contenues dans ledit ferrofluide soient entraînées au fond dudit container ;
   e) l'agitation de la suspension obtenue à l'étape d) ;
   f) la lecture à l'oeil et/ou par tout autre système de lecture approprié de la présence éventuelle d'agglutinats érythrocytaires dans le container.

2. Procédé selon la revendication 1, **caractérisé en ce que** ladite solution aqueuse de ferrofluide est sans agent tensio-actif.

3. Procédé selon la revendication 1 ou 2, **caractérisé en ce que** ledit ferrofluide est préparé à partir d'un mélange polyoxoanions de Fe(III) et d'au moins un métal M(II) de degré II d'oxydation choisi parmi les métaux de la première série des métaux de transition tels que Fe(II), Co(II), Mn(II), Cu(II) ou Ni(II).

4. Procédé selon l'une des revendications 1 à 3, **caractérisé en ce que** ledit ferrofluide est préparé à partir d'un mélange polyoxoanions de Fe(III) et Fe(II)).

5. Procédé selon l'une des revendications 1 à 4, **caractérisé en ce que** ledit ferrofluide est préparé à partir dudit mélange polyoxoanions associé à un cation.

6. Procédé selon la revendication 5, **caractérisé en ce que** ledit cation est choisi parmi $H^+$, $CH_3^+$, $N(CH_3)_4^+$, $N(C_2H_5)_4^+$.

7. Procédé selon l'une des revendications 1 à 6, **caractérisé en ce que** les sources de métaux de départ choisis pour Fe(III) et M(II) pour préparer ledit ferrofluide seront respectivement le chlorure ferrique et le chlorure ferreux.

8. Procédé selon l'une des revendications 1 à 7, **caractérisé en ce que** ledit ferrofluide est préparé à partir d'un mélange initial Fe(III) et de métal M(II) de rapport molaire compris entre 1 et 3.

9. Procédé selon les revendications 1 à 8, **caractérisé en ce que** ledit ferrofluide est préparé à partir d'un mélange initial Fe(III) et de métal M(II) de rapport molaire égal à $2 \pm 0,1$.

10. Procédé selon la revendication 1, **caractérisé en ce que** ledit ferrofluide est susceptible d'être obtenu par les procédés décrits aux exemples 1 à 8 de la demande de brevet français publiée sous le N° 2 461 521.

11. Procédé selon l'une des revendications 1 à 10, **caractérisé en ce que** ladite solution aqueuse de ferrofluide est préalablement diluée dans un tampon ou une solution physiologique.

12. Procédé selon l'une des revendications 1 à 10, **caractérisé en ce que** ladite solution aqueuse de ferrofluide est diluée de 0,25 à 10 % (v/v) dans ledit tampon ou ladite solution physiologique.

**13.** Procédé selon l'une des revendications 11 et 12, **caractérisé en ce que** ledit tampon est un tampon de basse force ionique.

**14.** Procédé selon l'une des revendications 1 à 13, **caractérisé en ce que** à l'étape a), ladite solution de ferrofluide est une solution de ferrofluide obtenu ou susceptible d'être obtenu à partir d'un mélange polyoxoanions de Fe(III) et d'au moins un métal M(II) de degré II d'oxydation qui a été préalablement diluée dans un tampon ou une solution physiologique dans une gamme comprise entre 0,2 et 2 % (v/v).

**15.** Procédé selon la revendication 14, **caractérisé en ce que** ladite solution de ferrofluide obtenu ou susceptible d'être obtenu à partir d'un mélange polyoxoanions de Fe(III) et d'au moins un métal M(II) de degré II d'oxydation a été préalablement diluée dans un tampon ou une solution physiologique dans une gamme comprise entre 0,25 et 0,75 % (v/v).

**16.** Procédé selon l'une des revendications 1 à 15, **caractérisé en ce qu'**avant l'étape b), ladite suspension d'érythrocytes contenus dans l'échantillon est préalablement soumise à l'action d'une enzyme protéasique.

**17.** Procédé selon la revendication 16, **caractérisé en ce que** ladite suspension d'érythrocytes est soumise à l'action de l'enzyme protéasique à la fin de l'étape a).

**18.** Procédé selon l'une des revendications 1 à 17, **caractérisé en ce que** les anticorps tests anti-antigène de groupe sanguin connu sont des anticorps agglutinants.

**19.** Procédé selon l'une des revendications 1 à 18, **caractérisé en ce que** l'étape d'incubation à la fin de l'étape b) est effectuée pendant un temps compris entre 5 et 15 min.

**20.** Procédé selon l'une des revendications 1 à 19, **caractérisé en ce que** l'étape c) d'agitation est effectuée pendant un temps compris entre 30 sec et 2 min 30 sec.

**21.** Procédé selon l'une des revendications 1 à 20, **caractérisé en ce que** l'étape d) d'application d'un champ magnétique audit container est réalisée au moyen d'un aimant situé à l'extérieur sous le container de telle manière que les particules de ferrofluide soient entraînées au fond dudit container selon un axe vertical.

**22.** Procédé selon la revendication 21, **caractérisé en ce que** l'étape d) ledit aimant est un aimant permanent de magnitude comprise entre 10 000 et 14 000 gauss.

**23.** Procédé selon la revendication 21 ou 22, **caractérisé en ce que** à l'étape d) le champ magnétique est appliqué pendant 2,5 min. à 10 min audit container.

**24.** Procédé selon l'une des revendications 1 à 23, **caractérisé en ce que** à l'étape e), l'agitation de la suspension avant lecture est effectuée en deux étapes d'agitation successives, la deuxième étant moins longue et moins vigoureuse que la première.

**25.** Procédé selon l'une des revendications 1 à 24, **caractérisé en ce que** à l'étape a), ladite suspension d'érythrocytes issus d'érythrocytes contenus dans l'échantillon est issue d'un culot d'érythrocytes obtenu après sédimentation d'un échantillon de sang total d'un individu.

**26.** Procédé selon l'une des revendications 1 à 25, **caractérisé en ce que** à l'étape b), ledit container est une cupule de microplaque.

**27.** Procédé selon la revendication 26, **caractérisé en ce que** à l'étape c), l'agitation est effectuée à l'aide d'un agitateur pour microplaque à une vitesse comprise entre 500 et 800 trs/min.

**28.** Procédé selon la revendication 26 ou 27, **caractérisé en ce que** à l'étape e), l'agitation est effectuée en 2 étapes à l'aide d'un agitateur pour microplaque, une première d'agitation pendant 1 à 2 min 30 sec à une vitesse comprise entre 800 et 1000 trs/min, et une deuxième agitation pendant 30 sec à 1 min à une vitesse comprise entre 350 et 550 trs/min.

**29.** Procédé selon l'une des revendications 26 à 28, **caractérisé en ce que** à l'étape b) :

- on met en contact dans ladite cupule de 40 µl à 50 µl de la suspension d'érythrocytes obtenue à l'étape a) avec des anticorps tests anti-antigène de groupe sanguin connu contenus sous forme desséchée au fond de la cupule.

**30.** Procédé selon l'une des revendications 1 à 29, pour le groupage sanguin ABO d'un échantillon de sang total issu d'un individu, **caractérisé en ce que** à l'étape b) :

- on met en contact dans autant de containers distincts la suspension d'érythrocytes issus de l'échantillon obtenue à l'étape a) avec un anticorps test anti-A et un anticorps test anti-B ; et
- on met en contact dans autant de containers distincts la suspension d'érythrocytes tests de groupe A et de groupe B obtenue à l'étape a) après mélange d'une suspension d'érythrocytes tests avec une solution aqueuse de ferrofluide, avec un échantillon de plasma ou de sérum de l'individu,

**caractérisé en ce que** à l'étape f) on évalue par lecture à l'oeil et/ou par tout autre système de lecture approprié la présence éventuelle d'agglutinats érythrocytaires dans chacun desdits containers, l'ensemble de ces lectures permettant de déterminer le groupe ABO de l'individu.

**31.** Procédé selon l'une des revendications 1 à 29, pour le phénotypage sanguin, hors épreuve de Simonin, d'un échantillon de sang total issu d'un individu, **caractérisé en ce que** à l'étape b) :

- on met en contact dans autant de containers distincts la suspension d'érythrocytes issus de l'échantillon obtenue à l'étape a) avec un anticorps test spécifique de l'antigène de groupe sanguin dont on cherche à déterminer la présence ou l'absence sur les érythrocytes de l'échantillon, chaque container contenant une seule spécificité d'anticorps,

**caractérisé en ce que** à l'étape f) on évalue par lecture à l'oeil et/ou par tout autre système de lecture approprié la présence éventuelle d'agglutinats érythrocytaires dans chacun desdits containers, l'ensemble de ces lectures permettant de déterminer le phénotype de l'individu pour les antigènes de groupe sanguin recherchés.

**32.** Procédé selon l'une des revendications 1 à 30, pour le phénotypage sanguin, épreuve de Simonin comprise, d'un échantillon de sang total issu d'un individu, **caractérisé en ce que** à l'étape b) :

- on met en contact dans autant de containers distincts la suspension d'érythrocytes issus de l'échantillon obtenue à l'étape a) avec un anticorps test anti-A et un anticorps test anti-B, et
- on met en contact dans autant d'autres containers distincts la suspension d'érythrocytes issus de l'échantillon obtenue à l'étape a) avec un anticorps test spécifique de l'antigène de groupe sanguin, hors antigène A et B, dont on cherche à déterminer la présence ou l'absence sur les érythrocytes de l'échantillon, chaque autre container contenant une seule spécificité d'anticorps ; et
- on met en contact dans autant de containers distincts la suspension d'érythrocytes tests de groupe A et de groupe B obtenue à l'étape a) après mélange d'une suspension d'érythrocytes tests avec une solution aqueuse de ferrofluide, avec un échantillon de plasma ou de sérum de l'individu,

**caractérisé en ce que** à l'étape f) on évalue par lecture à l'oeil et/ou par tout autre système de lecture approprié la présence éventuelle d'agglutinats érythrocytaires dans chacun desdits containers, l'ensemble de ces lectures permettant de déterminer le groupe ABO de l'individu et de déterminer le phénotype de l'individu pour les autres antigènes de groupe sanguin recherchés.

**33.** Procédé selon la revendication 30 ou 32, **caractérisé en ce qu'**à l'étape b), l'échantillon de plasma ou de sérum de l'individu est mis en contact avec également une suspension d'érythrocytes test de groupe O obtenue à l'étape a).

**34.** Dispositif pour la détermination par réaction d'agglutination d'érythrocytes de la présence d'un antigène du groupe sanguin à la surface d'un érythrocyte d'un échantillon, **caractérisé en ce qu'**il comprend :

a) un container contenant une solution de ferrofluide telle que définie dans les revendications 1 à 13 ;
b) un container contenant une suspension d'érythrocytes de l'échantillon à tester ;
c) un ou plusieurs containers à réaction contenant un anticorps test anti-antigène de groupe sanguin ;
d) au moins un aimant ou un ensemble d'aimants pouvant être disposé à l'extérieur dudit ou desdits containers et sous ledit ou lesdits containers ;

e) un système d'agitation dudit ou desdits containers.

**35.** Dispositif selon la revendication 34, **caractérisé en ce que** ledit container à réaction est une cupule de microplaque.

**36.** Dispositif selon la revendication 34 ou 35, pour le groupage sanguin ABO d'un échantillon de sang total issu d'un individu, **caractérisé en ce que** :

- lesdits containers contenant une suspension d'érythrocytes tests de groupe connu contiennent distinctement une suspension d'érythrocytes tests de groupe A, une suspension d'érythrocytes tests de groupe B; et
- lesdits containers contenant un anticorps test anti-antigène de groupe sanguin contiennent distinctement un anticorps anti-A et un anticorps anti-B.

**37.** Dispositif selon la revendication 34 ou 35, pour le phénotypage sanguin, hors épreuve de Simonin, d'un échantillon de sang total issu d'un individu, **caractérisé en ce que** :

- lesdits containers contiennent distinctement un anticorps test spécifique de l'antigène de groupe sanguin dont on cherche à déterminer la présence ou l'absence sur les érythrocytes de l'échantillon.

**38.** Dispositif selon la revendication 34 ou 35, pour le phénotypage sanguin, épreuve de Simonin comprise, d'un échantillon de sang total issu d'un individu, **caractérisé en ce que** :

- lesdits containers contenant une suspension d'érythrocytes tests de groupe connu contiennent distinctement une suspension d'érythrocytes tests de groupe A, une suspension d'érythrocytes tests de groupe B; et
- lesdits containers contenant un anticorps test anti-antigène de groupe sanguin contiennent distinctement un anticorps anti-A, un anticorps anti-B et un anticorps test spécifique de l'antigène de groupe sanguin, hors ABO, dont on cherche à déterminer la présence ou l'absence sur les érythrocytes de l'échantillon.

**39.** Kit pour la détermination par réaction d'agglutination d'érythrocytes de la présence d'un antigène du groupe sanguin à la surface d'un érythrocyte d'un échantillon, **caractérisé en ce qu'**il comprend :

a) un ou plusieurs containers à réaction contenant distinctement un anticorps test anti-antigène de groupe sanguin ;
b) une solution de ferrofluide telle que définie dans l'une des revendications 1 à 13 ; et
c) un ou plusieurs aimants pouvant être disposés à l'extérieur sous le ou les containers à réaction.

**40.** Kit selon la revendication 39 pour le groupage sanguin ABO d'un échantillon de sang total issu d'un individu, **caractérisé en ce que** :

- lesdits containers contiennent en outre une suspension d'érythrocytes tests de groupe de groupe A et une suspension d'érythrocytes tests de groupe B; et
- lesdits containers contenant un anticorps test anti-antigène de groupe sanguin contiennent distinctement un anticorps anti-A et un anticorps anti-B.

**41.** Kit selon la revendication 40, pour le phénotypage sanguin, épreuve de Simonin comprise, d'un échantillon de sang total issu d'un individu, **caractérisé en ce qu'**il comprend en outre au moins autant de containers contenant distinctement un anticorps test spécifique de l'antigène de groupe sanguin, autre que l'antigène A et B, dont on cherche à déterminer la présence ou l'absence sur les érythrocytes de l'échantillon à phénotyper.

**42.** Kit selon l'une des revendications 39 à 41, **caractérisé en ce que** lesdits anticorps test anti-antigène de groupe sanguin contenus dans lesdits containers sont sous forme desséchée.

**43.** Kit selon l'une des revendications 39 à 42, **caractérisé en ce que** lesdits containers sont des cupules de microplaque.

**Patentansprüche**

**1.** Blutgruppen-Phänotypisierungsverfahren, in welchem Verfahren man durch Agglutinationsreaktion von Erythrozyten das Vorhandensein eines Blutgruppen-Antigens auf der Oberfläche eines Erythrozyten einer Probe bestimmt, **dadurch gekennzeichnet, dass** es die folgenden Schritte umfasst:

a) das Mischen einer Suspension von Erythrozyten, die in der Probe enthalten sind, mit einer wässrigen Lösung von Ferrofluid, das ausgehend von einer Mischung von Polyoxoanionen von Fe(III) und von mindestens einem Metall M(II) der Oxidationsstufe II erhalten wird oder erhalten werden kann, derart, dass eine homogene Suspension der Erythrozyten in der Ferrofluid-Lösung erhalten wird;

b) das Inkontaktbringen der in dem Schritt a) erhaltenen Suspension von Erythrozyten mit einem gegen ein bekanntes Blutgruppen-Antigen gerichteten Test-Antikörper in einem Behälter, wobei diesem Schritt ein Inkubationsschritt folgt;

c) die Bewegung der in dem Schritt b) nach Inkubation erhaltenen Suspension;

d) die Anwendung eines Magnetfelds an dem Behälter derart, dass die in dem Ferrofluid enthaltenen magnetischen Teilchen an den Boden des Behälters gezogen werden;

e) die Bewegung der in dem Schritt d) erhaltenen Suspension;

f) das Ablesen des etwaigen Vorhandenseins von Erythrozyten enthaltenden Agglutinationsprodukten in dem Behälter mit dem Auge und/oder durch ein jegliches anderes geeignetes Ablesesystem.

2. Verfahren nach Anspruch 1, **dadurch gekennzeichnet, dass** die wässrige Ferrofluid-Lösung kein grenzflächenaktives Mittel aufweist.

3. Verfahren nach Anspruch 1 oder 2, **dadurch gekennzeichnet, dass** das Ferrofluid ausgehend von einer Mischung von Polyoxoanionen von Fe(III) und von mindestens einem Metall M(II) der Oxidationsstufe II, ausgewählt unter den Metallen der ersten Reihe der Übergangsmetalle, wie Fe(II), Co(II), Mn(II), Cu(II) oder Ni(II), hergestellt wird.

4. Verfahren nach einem der Ansprüche 1 bis 3, **dadurch gekennzeichnet, dass** das Ferrofluid ausgehend von einer Mischung von Polyoxoanionen von Fe(III) und Fe(II) hergestellt wird.

5. Verfahren nach einem der Ansprüche 1 bis 4, **dadurch gekennzeichnet, dass** das Ferrofluid ausgehend von der Mischung von Polyoxoanionen, die mit einem Kation assoziiert ist, hergestellt wird.

6. Verfahren nach Anspruch 5, **dadurch gekennzeichnet, dass** das Kation unter $H^+$, $CH_3^+$, $N(CH_3)_4^+$, $N(C_2H_5)_4^+$ ausgewählt ist.

7. Verfahren nach einem der Ansprüche 1 bis 6, **dadurch gekennzeichnet, dass** die Quellen von Ausgangsmetall, die für Fe(III) und M(II) ausgewählt werden, um das Ferrofluid herzustellen, Eisen(III)-chlorid bzw. Eisen(II)-chlorid sind.

8. Verfahren nach einem der Ansprüche 1 bis 7, **dadurch gekennzeichnet, dass** das Ferrofluid ausgehend von einer anfänglichen Mischung von Fe(III) und von Metall M(II) mit einem Molverhältnis zwischen 1 und 3 eingeschlossen hergestellt wird.

9. Verfahren nach den Ansprüchen 1 bis 8, **dadurch gekennzeichnet, dass** das Ferrofluid ausgehend von einer anfänglichen Mischung von Fe(III) und von Metall (II) mit einem Molverhältnis gleich $2 \pm 0,1$ hergestellt wird.

10. Verfahren nach Anspruch 1, **dadurch gekennzeichnet, dass** das Ferrofluid durch die in den Beispielen 1 bis 8 der unter der Nr. 2 461 521 veröffentlichten französischen Patentanmeldung beschriebenen Verfahren erhalten werden kann.

11. Verfahren nach einem der Ansprüche 1 bis 10, **dadurch gekennzeichnet, dass** die wässrige Lösung von Ferrofluid vorab in einem Puffer oder einer physiologischen Lösung verdünnt wird.

12. Verfahren nach einem der Ansprüche 1 bis 10, **dadurch gekennzeichnet, dass** die wässrige Lösung von Ferrofluid von 0,25 bis 10% (Vol./Vol.) in dem Puffer oder der physiologischen Lösung verdünnt wird.

13. Verfahren nach einem der Ansprüche 11 und 12, **dadurch gekennzeichnet, dass** der Puffer ein Puffer von geringer Ionenstärke ist.

14. Verfahren nach einem der Ansprüche 1 bis 13, **dadurch gekennzeichnet, dass** in dem Schritt a) die Lösung von Ferrofluid eine Ferrofluid-Lösung ist, die ausgehend von einer Mischung von Polyoxoanionen von Fe(III) und von mindestens einem Metall M(II) der Oxidationsstufe II, die vorab in einem Puffer oder einer physiologischen Lösung in einem Bereich zwischen 0,2 und 2% (Vol./Vol.) eingeschlossen verdünnt worden ist, erhalten wird oder erhalten werden kann.

15. Verfahren nach Anspruch 14, **dadurch gekennzeichnet, dass** die Lösung von Ferrofluid, die ausgehend von einer Mischung von Polyoxoanionen von Fe(III) und von mindestens einem Metall M(II) der Oxidationsstufe II erhalten wird oder erhalten werden kann, vorab in einem Puffer oder einer physiologischen Lösung in einem Bereich zwischen 0,25 und 0,75% (Vol./Vol.) eingeschlossen verdünnt worden ist.

16. Verfahren nach einem der Ansprüche 1 bis 15, **dadurch gekennzeichnet, dass** vor dem Schritt b) die Suspension von Erythrozyten, die in der Probe enthalten sind, vorab der Wirkung eines Protease-Enzyms unterworfen wird.

17. Verfahren nach Anspruch 16, **dadurch gekennzeichnet, dass** die Suspension von Erythrozyten der Wirkung des Protease-Enzyms am Ende des Schritts a) unterworfen wird.

18. Verfahren nach einem der Ansprüche 1 bis 17, **dadurch gekennzeichnet, dass** die gegen ein bekanntes Blutgruppen-Antigen gerichteten Test-Antikörper agglutinierende Antikörper sind.

19. Verfahren nach einem der Ansprüche 1 bis 18, **dadurch gekennzeichnet, dass** der Inkubationsschritt am Ende des Schritts b) während einer Zeitspanne zwischen 5 und 15 Minuten eingeschlossen ausgeführt wird.

20. Verfahren nach einem der Ansprüche 1 bis 19, **dadurch gekennzeichnet, dass** der Bewegungsschritt c) während einer Zeitspanne zwischen 30 Sekunden und 2 Minuten 30 Sekunden eingeschlossen ausgeführt wird.

21. Verfahren nach einem der Ansprüche 1 bis 20, **dadurch gekennzeichnet, dass** der Schritt d) der Anwendung eines Magnetfelds an dem Behälter ausgeführt wird mittels eines Magneten, der sich auf der Außenseite unter dem Behälter befindet derart, dass die Teilchen von Ferrofluid auf den Boden des Behälters gemäß einer vertikalen Achse gezogen werden.

22. Verfahren nach Anspruch 21, **dadurch gekennzeichnet, dass** in dem Schritt d) der Magnet ein Permanentmagnet einer Stärke zwischen 10000 und 14000 Gauss eingeschlossen ist.

23. Verfahren nach Anspruch 21 oder 22, **dadurch gekennzeichnet, dass** in dem Schritt d) das Magnetfeld während 2,5 Minuten bis 10 Minuten an dem Behälter angewandt wird.

24. Verfahren nach einem der Ansprüche 1 bis 23, **dadurch gekennzeichnet, dass** in dem Schritt e) die Bewegung der Suspension vor dem Ablesen in zwei aufeinanderfolgenden Bewegungsschritten ausgeführt wird, wobei der zweite weniger lang und weniger kräftig als der erste ist.

25. Verfahren nach einem der Ansprüche 1 bis 24, **dadurch gekennzeichnet, dass** in dem Schritt a) die Suspension von Erythrozyten, die von Erythrozyten stammen, die in der Probe enthalten sind, von einem Bodensatz von Erythrozyten, der nach Sedimentation einer Vollblutprobe eines Individuums erhalten worden ist, stammt.

26. Verfahren nach einem der Ansprüche 1 bis 25, **dadurch gekennzeichnet, dass** in dem Schritt b) der Behälter eine Mikroplatten-Vertiefung (Mikroplatten-Well) ist.

27. Verfahren nach Anspruch 26, **dadurch gekennzeichnet, dass** in dem Schritt c) die Bewegung mit Hilfe eines Mikroplatten-Schüttlers mit einer Geschwindigkeit zwischen 500 und 800 Umdrehungen/min eingeschlossen ausgeführt wird.

28. Verfahren nach Anspruch 26 oder 27, **dadurch gekennzeichnet, dass** in dem Schritt e) die Bewegung in 2 Schritten mit Hilfe eines Mikroplatten-Schüttlers ausgeführt wird, eine erste Bewegung während 1 bis 2 Minuten 30 Sekunden bei einer Geschwindigkeit zwischen 800 und 1000 Umdrehungen/min eingeschlossen und eine zweite Bewegung während 30 Sekunden bis 1 Minute bei einer Geschwindigkeit zwischen 350 und 550 Umdrehungen/min eingeschlossen.

29. Verfahren nach einem der Ansprüche 26 bis 28, **dadurch gekennzeichnet, dass** in dem Schritt b):

- man in der Vertiefung (Well) 40 μl bis 50 μl der in dem Schritt a) erhaltenen Erythrozytensuspension mit gegen ein bekanntes Blutgruppen-Antigen gerichteten Test-Antikörpern, die in getrockneter Form am Boden der Vertiefung (Well) enthalten sind, in Kontakt bringt.

**30.** Verfahren nach einem der Ansprüche 1 bis 29 für die ABO-Blutgruppenbestimmung einer von einem Individuum stammenden Vollblutprobe, **dadurch gekennzeichnet, dass** in dem Schritt b):

- man in ebenso vielen unterschiedlichen Behältern die Suspension von aus der Probe stammenden Erythrozyten, die in dem Schritt a) erhalten worden ist, mit einem anti-A-Test-Antikörper und einem anti-B-Test-Antikörper in Kontakt bringt; und
- man in ebenso vielen unterschiedlichen Behältern die in dem Schritt a) erhaltene Suspension von Test-Erythrozyten von Gruppe A und von Gruppe B nach Mischen einer Suspension von Test-Erythrozyten mit einer wässrigen Lösung von Ferrofluid mit einer Plasma- oder Serumprobe des Individuums in Kontakt bringt, **dadurch gekennzeichnet, dass** man in dem Schritt f) das etwaige Vorhandensein von Erythrozyten enthaltenden Agglutinationsprodukten in jedem der Behälter durch Ablesen mit dem Auge und/oder durch ein jegliches anderes geeignetes Ablesesystem auswertet, wobei die Gesamtheit dieser Ablesungen erlaubt, die ABO-Gruppe des Individuums zu bestimmen.

**31.** Verfahren nach einem der Ansprüche 1 bis 29 für die Blut-Phänotypisierung, mit Ausnahme des Simonin-Tests ("Epreuve de Simonin"), einer von einem Individuum stammenden Vollblutprobe, **dadurch gekennzeichnet, dass** in dem Schritt b):

- man in ebenso vielen unterschiedlichen Behältern die in dem Schritt a) erhaltene Suspension von aus der Probe stammenden Erythrozyten mit einem für das Blutgruppen-Antigen, dessen Vorhandensein oder Abwesenheit auf den Erythrozyten der Probe man zu bestimmen strebt, spezifischen Test-Antikörper in Kontakt bringt, wobei jeder Behälter eine einzige Antikörper-Spezifität enthält, **dadurch gekennzeichnet**, man in dem Schritt f) das etwaige Vorhandensein von Erythrozyten enthaltenden Agglutinationsprodukten in jedem der Behälter durch Ablesen mit dem Auge und/oder durch ein jegliches anderes geeignetes Ablesesystem auswertet, wobei die Gesamtheit dieser Ablesungen erlaubt, den Phänotyp des Individuums für die untersuchten Blutgruppen-Antigene zu bestimmen.

**32.** Verfahren nach einem der Ansprüche 1 bis 30 für die Blut-Phänotypisierung, einschließlich des Simonin-Tests ("Epreuve de Simonin"), einer von einem Individuum stammenden Vollblutprobe, **dadurch gekennzeichnet, dass** in dem Schritt b):

- man in ebenso vielen unterschiedlichen Behältern die in dem Schritt a) erhaltene Suspension von aus der Probe stammenden Erythrozyten mit einem anti-A-Test-Antikörper und einem anti-B-Test-Antikörper in Kontakt bringt, und
- man in ebenso vielen anderen unterschiedlichen Behältern die in dem Schritt a) erhaltene Suspension von aus der Probe stammenden Erythrozyten mit einem für das Blutgruppen-Antigen, außer dem A- und B-Antigen, dessen Vorhandensein oder Abwesenheit auf den Erythrozyten der Probe man zu bestimmen strebt, spezifischen Test-Antikörper in Kontakt bringt, wobei jeder andere Behälter eine einzige Antikörper-Spezifität enthält; und
- man in ebenso vielen unterschiedlichen Behältern die in dem Schritt a) erhaltene Suspension von Test-Erythrozyten von Gruppe A und von Gruppe B nach Mischen einer Suspension von Test-Erythrozyten mit einer wässrigen Ferrofluid-Lösung mit einer Plasma- oder Serumprobe des Individuums in Kontakt bringt, **dadurch gekennzeichnet, dass** man in dem Schritt f) das etwaige Vorhandensein von Erythrozyten enthaltenden Agglutinationsprodukten in jedem der Behälter durch Ablesen mit dem Auge und/oder durch ein jegliches anderes geeignetes Ablesesystem auswertet, wobei die Gesamtheit dieser Ablesungen erlaubt, die ABO-Gruppe des Individuums zu bestimmen und den Phänotyp des Individuums für die anderen untersuchten Blutgruppen-Antigene zu bestimmen.

**33.** Verfahren nach Anspruch 30 oder 32, **dadurch gekennzeichnet, dass** in dem Schritt b) die Plasma- oder Serumprobe des Individuums gleichfalls mit einer Suspension von Test-Erythrozyten von Gruppe O, die in dem Schritt a) erhalten worden ist, in Kontakt gebracht wird.

**34.** Anordnung für die Bestimmung des Vorhandenseins eines Blutgruppen-Antigens auf der Oberfläche eines Erythrozyten einer Probe durch Agglutinationsreaktion von Erythrozyten, **dadurch gekennzeichnet, dass** sie umfasst:

a) einen Behälter, welcher eine Ferrofluid-Lösung, wie in den Ansprüchen 1 bis 13 definiert, enthält;

b) einen Behälter, welcher eine Suspension von Erythrozyten der zu testenden Probe enthält;

c) einen oder mehrere Reaktionsbehälter, welche(r) einen anti-Blutgruppen-Antigen-Test-Antikörper enthält bzw. enthalten;

d) mindestens einen Magneten oder eine Gruppe von Magneten, die auf der Außenseite des oder der Behälter (s) und unter dem oder den Behälter(n) angeordnet sein können;

e) ein System zum Bewegen des oder der Behälter.

35. Anordnung nach Anspruch 34, **dadurch gekennzeichnet, dass** der Reaktionsbehälter eine Mikroplatten-Vertiefung (-Well) ist.

36. Anordnung nach Anspruch 34 oder 35 für die ABO-Blutgruppenbestimmung einer von einem Individuum stammenden Vollblutprobe, **dadurch gekennzeichnet, dass**:

   - die Behälter, welche eine Suspension von Test-Erythrozyten einer bekannten Gruppe enthalten, voneinander verschieden eine Suspension von Test-Erythrozyten von Gruppe A, eine Suspension von Test-Erythrozyten von Gruppe B enthalten; und
   - die Behälter, welche einen anti-Blutgruppen-Antigen-Test-Antikörper enthalten, voneinander verschieden einen anti-A-Antikörper und einen anti-B-Antikörper enthalten.

37. Anordnung nach Anspruch 34 oder 35 für die Blut-Phänotypisierung, mit Ausnahme des Simonin-Tests ("Epreuve de Simonin"), einer von einem Individuum stammenden Vollblutprobe, **dadurch gekennzeichnet, dass**:

   - die Behälter voneinander verschieden einen für das Blutgruppen-Antigen, dessen Vorhandensein oder Abwesenheit auf den Erythrozyten der Probe man zu bestimmen strebt, spezifischen Test-Antikörper enthalten.

38. Anordnung nach Anspruch 34 oder 35 für die Blut-Phänotypisierung, einschließlich des Simonin-Tests ("Epreuve de Simonin"), einer von einem Individuum stammenden Vollblutprobe, **dadurch gekennzeichnet, dass**:

   - die Behälter, welche eine Suspension von Test-Erythrozyten einer bekannten Gruppe enthalten, voneinander verschieden eine Suspension von Test-Erythrozyten von Gruppe A, eine Suspension von Test-Erythrozyten von Gruppe B enthalten; und
   - die Behälter, welche einen anti-Blutgruppen-Antigen-Test-Antikörper enthalten, voneinander verschieden einen anti-A-Antikörper, einen anti-B-Antikörper und einen für das Blutgruppen-Antigen, außer ABO, dessen Vorhandensein oder Abwesenheit auf den Erythrozyten der Probe man zu bestimmen strebt, spezifischen Test-Antikörper enthalten.

39. Kit für die Bestimmung des Vorhandenseins eines Blutgruppen-Antigens auf der Oberfläche eines Erythrozyten einer Probe durch Agglutinationsreaktion von Erythrozyten, **dadurch gekennzeichnet, dass** er umfasst:

   a) einen oder mehrere Reaktionsbehälter, welche(r) voneinander verschieden einen anti-Blutgruppen-Antigen-Test-Antikörper enthält bzw. enthalten;

   b) eine Ferrofluid-Lösung, wie in einem der Ansprüche 1 bis 13 definiert, und;

   c) einen oder mehrere Magneten, die auf der Außenseite unter dem oder den Reaktionsbehälter(n) angeordnet sein können.

40. Kit nach Anspruch 39 für die ABO-Blutgruppenbestimmung einer von einem Individuum stammenden Vollblutprobe, **dadurch gekennzeichnet, dass**:

   - die Behälter außerdem eine Suspension von Test-Erythrozyten von Gruppe von Gruppe A und eine Suspension von Test-Erythrozyten von Gruppe B enthalten; und
   - die Behälter, welche einen anti-Blutgruppen-Antigen-Test-Antikörper enthalten, voneinander verschieden einen anti-A-Antikörper und einen anti-B-Antikörper enthalten.

41. Kit nach Anspruch 40 für die Blut-Phänotypisierung, einschließlich des Simonin-Tests ("Epreuve de Simonin"), einer von einem Individuum stammenden Vollblutprobe, **dadurch gekennzeichnet, dass** er außerdem mindestens ebenso viele Behälter, welche voneinander verschieden einen für das Blutgruppen-Antigen, außer dem A- und B-Antigen, dessen Vorhandensein oder Abwesenheit auf den Erythrozyten der zu phänotypisierenden Probe man zu bestimmen strebt, spezifischen Test-Antikörper enthalten, umfasst.

42. Kit nach einem der Ansprüche 39 bis 41, **dadurch gekennzeichnet, dass** die in den Behältern enthaltenen anti-Blutgruppen-Antigen-Test-Antikörper in getrockneter Form vorliegen.

43. Kit nach einem der Ansprüche 39 bis 42, **dadurch gekennzeichnet, dass** die Behälter Mikroplatten-Vertiefungen (-Wells) sind.

**Claims**

1. A method of phenotyping blood for determining, by an erythrocyte agglutination reaction, the presence of a blood-group antigen on the surface of an erythrocyte of a sample, **characterized in that** it is comprised of the following steps:

    a) the mixture of a suspension of erythrocytes contained in the sample with an aqueous ferrofluid solution obtained or likely to be obtained from a mixture of polyoxoanions of Fe(III) and of at least one M(II) metal of oxidation state II, so as to obtain a homogeneous suspension of the aforesaid erythrocytes in the ferrofluid solution;
    b) the placing in contact in a container of the suspension of erythrocytes obtained in step a) with an anti-antigen test antibody of a known blood group, this step being followed by an incubation step;
    c) the shaking of the suspension obtained in step b) after incubation;
    d) the application of a magnetic field to said container in such a way that the magnetic particles contained in the aforesaid ferrofluid are pulled to the bottom of the aforesaid container;
    e) the shaking of the suspension obtained in step d);
    f) the reading by the naked eye and/or by any other suitable reading system of the possible presence of erythrocyte agglutinates in the container.

2. A method according to claim 1, **characterized in that** the aforementioned aqueous ferrofluid solution is surfactant-free.

3. A method according to claim 1 or 2, **characterized in that** the aforementioned ferrofluid is prepared from a mixture of polyoxoanions of Fe(III) and of at least one M(II) metal of oxidation state II selected among the metals of the first series of transition metals such as Fe(II), Co(II), Mn(II), Cu(II) or Ni(II).

4. A method according to one of the claims 1 to 3, **characterized in that** the aforementioned ferrofluid is prepared from a mixture of polyoxoanions of Fe (III) and Fe (II).

5. A method according to one of the claims 1 to 4, **characterized in that** the aforementioned ferrofluid is prepared from the aforesaid mixture of polyoxoanions associated with a cation.

6. A method according to claim 5, **characterized in that** the aforementioned cation is selected among $H^+$, $CH_3^+$, $N(CH_3)_4^+$, $N(C_2H_5)_4^+$.

7. A method according to one of the claims 1 to 6, **characterized in that** the sources of starting metals chosen for Fe (III) and M(II) for preparing the aforementioned ferrofluid will be ferric chloride and ferrous chloride, respectively.

8. A method according to one of the claims 1 to 7, **characterized in that** the aforementioned ferrofluid is prepared from an initial mixture of Fe(III) and of M(II) metal of a molar ratio between 1 and 3.

9. A method according to claims 1 to 8, **characterized in that** the aforementioned ferrofluid is prepared from an initial mixture of Fe(III) and of M(II) metal of a molar ratio equal to $2 \pm 0.1$.

10. A method according to claim 1, **characterized in that** the aforementioned ferrofluid is likely to be obtained by the methods disclosed in examples 1 to 8 of the French patent application published under the number 2 461 521.

11. A method according to one of the claims 1 to 10, **characterized in that** the aforementioned aqueous ferrofluid solution is diluted beforehand in a buffer or in a physiological solution.

12. A method according to one of the claims 1 to 10, **characterized in that** the aforementioned aqueous ferrofluid solution is diluted to between 0.25% and 10% (v/v) in the aforementioned buffer or in the aforementioned physiological solution.

13. A method according to one of claims 11 and 12, **characterized in that** the aforementioned buffer is a low ionic strength solution buffer.

14. A method according to one of the claims 1 to 13, **characterized in that** in step a), the aforementioned ferrofluid solution is a ferrofluid solution obtained or likely to be obtained from a mixture of polyoxoanions of Fe(III) and of at least one M(II) metal of oxidation state II which was diluted beforehand in a buffer or in a physiological solution in a range between 0.2% and 2% (v/v).

15. A method according to claim 14, **characterized in that** the aforementioned ferrofluid solution obtained or likely to be obtained from a mixture of polyoxoanions of Fe(III) and of at least one M(II) metal of oxidation state II was diluted beforehand in a buffer or a physiological solution in a range between 0.25% and 0.75% (v/v).

16. A method according to one of the claims 1 to 15, **characterized in that** before step b), the aforementioned suspension of erythrocytes contained in the sample is subjected beforehand to the action of a protease enzyme.

17. A method according to claim 16, **characterized in that** the aforementioned suspension of erythrocytes is subjected to the action of the protease enzyme at the end of step a).

18. A method according to one of the claims 1 to 17, **characterized in that** the anti-antigen test antibodies of a known blood group are agglutinating antibodies.

19. A method according to one of the claims 1 to 18, **characterized in that** the incubation step at the end of step b) is carried out for a period of time between 5 and 15 minutes.

20. A method according to one of the claims 1 to 19, **characterized in that** shaking step c) is carried out for a period of time between 30 seconds and 2.5 minutes.

21. A method according to one of the claims 1 to 20, **characterized in that** step d), application of a magnetic field to said container, is carried out by means of a magnet located outside and beneath the container in such a way that the particles of ferrofluid are pulled to the bottom of the aforesaid container according to a vertical axis.

22. A method according to claim 21, **characterized in that** in step d) the aforementioned magnet is a permanent magnet of a strength between 10,000 and 14,000 gauss.

23. A method according to claim 21 or 22, **characterized in that** in step d) the magnetic field is applied to said container for 2.5 minutes to 10 minutes.

24. A method according to one of the claims 1 to 23, **characterized in that** step e), the shaking of the suspension before reading, is carried out in two successive shaking steps, the second being shorter in time and less vigorous than the first.

25. A method according to one of the claims 1 to 24, **characterized in that** in step a) the aforementioned suspension of erythrocytes from erythrocytes contained in the sample results from a pellet of erythrocytes obtained after sedimentation of a sample of total blood of an individual.

26. A method according to one of the claims 1 to 25, **characterized in that** in step b) the aforementioned container is a microplate well.

27. A method according to claim 26, **characterized in that** in step c) shaking is carried out using a microplate shaker at a speed between 500 rpm and 800 rpm.

28. A method according to claim 26 or 27, **characterized in that** in step e) shaking is carried out in 2 steps using a microplate shaker, a first shaking for 1 minute to 2.5 minutes at a speed between 800 rpm and 1,000 rpm, and the second shaking for 30 seconds to 1 minute at a speed between 350 rpm and 550 rpm.

29. A method according to one of the claims 26 to 28, **characterized in that** in step b):

- 40 µl to 50 µl of the suspension of erythrocytes obtained in step a) are placed in contact in said well with the anti-antigen test antibodies of a known blood group contained in desiccated form at the bottom of the well.

30. A method according to one of the claims 1 to 29 for the ABO blood grouping of a sample of total blood from an individual, **characterized in that** in step b) :

- the suspension of erythrocytes from the sample obtained in step a) is placed in contact in distinct containers with an anti-A test antibody and an anti-B test antibody; and
- a sample of plasma or serum from the individual is placed in contact in distinct containers with a suspension of test erythrocytes of group A and of group B obtained in step a) after mixture of a suspension of test erythrocytes with an aqueous ferrofluid solution,

**characterized in that** in step f) the possible presence of erythrocyte agglutinates in each of the aforesaid containers is evaluated by reading with the naked eye and/or any other suitable reading system, the whole of these readings making it possible to determine the individual's ABO group.

31. A method according to one of the claims 1 to 29 for blood phenotyping, excluding the Simonin test, of a sample of total blood from an individual, **characterized in that** in step b):

- the suspension of erythrocytes from the sample obtained in step a) is placed in contact in distinct containers with a test antibody specific for the blood-group antigens whose presence or absence on the erythrocytes of the sample is sought to be determined, each container containing only one antibody specificity,

**characterized in that** in step f) the possible presence of erythrocyte agglutinates in each of the aforesaid containers is evaluated by reading with the naked eye and/or any other suitable reading system, the whole of these readings making it possible to determine the phenotype of the individual for the antigens of the blood group sought.

32. A method according to one of the claims 1 to 30 for blood phenotyping, Simonin test included, of a sample of total blood from an individual, **characterized in that** in step b):

- the suspension of erythrocytes from the sample obtained in step a) is placed in contact in distinct containers with an anti-A test antibody and an anti-B test antibody, and
- the suspension of erythrocytes from the sample obtained in step a) is placed in contact in other distinct containers with a test antibody specific for the blood-group antigen, except antigen A and B, whose presence or absence on the erythrocytes of the sample is sought to be determined, each container containing only one antibody specificity; and
- a sample of plasma or serum from the individual is placed in contact in other distinct containers with a suspension of test erythrocytes of group A and of group B obtained in step a) after mixture of a suspension of test erythrocytes with an aqueous ferrofluid solution,

**characterized in that** in step f) the possible presence of erythrocyte agglutinates in each of the aforesaid containers is evaluated by reading with the naked eye and/or any other suitable reading system, the whole of these readings making it possible to determine the ABO group of the individual and to determine the phenotype of the individual for the other antigens of the blood group sought.

33. A method according to claim 30 or 32, **characterized in that** in step b), the sample of plasma or serum from the individual is placed in contact also with a suspension of test erythrocytes of group O obtained after step a).

34. A device for determining, by an erythrocyte agglutination reaction, the presence of a blood-group antigen on the surface of an erythrocyte of a sample, **characterized in that** it is comprised of:

a) a container containing a ferrofluid solution such as defined in claims 1 to 13;
b) a container containing a suspension of erythrocytes of the sample to be tested;
c) one or more reaction containers containing an anti-antigen blood-group test antibody;
d) at least one magnet or an assembly of magnets which can be located outside and beneath said container or containers;

e) a shaking system for the aforesaid container or containers.

**35.** A device according to claim 34, **characterized in that** the aforementioned reaction container is a microplate well.

**36.** A device according to claim 34 or 35 for the ABO blood grouping of a sample of total blood from an individual, **characterized in that**:

- the aforementioned containers containing a suspension of test erythrocytes of a known group distinctly contain a suspension of test erythrocytes of group A, a suspension of test erythrocytes of group B; and
- the aforementioned containers containing an anti-antigen blood-group test antibody distinctly contain an anti-A antibody and an anti-B antibody.

**37.** A device according to claim 34 or 35 for blood phenotyping, excluding the Simonin test, of a sample of total blood from an individual, **characterized in that**:

- the aforementioned containers distinctly contain a test antibody specific for the blood-group antigens whose presence or absence on the erythrocytes of the sample is sought.

**38.** A device according to claim 34 or 35 for blood phenotyping, including the Simonin test, of a sample of total blood from an individual, **characterized in that**:

- the aforementioned containers containing a suspension of test erythrocytes of a known group distinctly contain a suspension of test erythrocytes of group A, a suspension of test erythrocytes of group B; and
- the aforementioned containers containing an anti-antigen blood-group test antibody distinctly contain an anti-A antibody, an anti-B antibody and a test antibody specific for the blood-group antigens, except ABO, whose presence or absence on the erythrocytes of the sample is sought to be determined.

**39.** A kit for determining, by an erythrocyte agglutination reaction, the presence of an antigen of the blood group on the surface of an erythrocyte of a sample, **characterized in that** it is comprised of:

a) one or more reaction containers distinctly containing an anti-antigen blood-group test antibody;
b) a ferrofluid solution as defined in one of claims 1 to 13; and
c) one or more magnets which can be placed outside and beneath the reaction containers.

**40.** A kit according to claim 39 for the ABO blood grouping of a sample of total blood from an individual, **characterized in that**:

- the aforementioned containers contain in addition a suspension of test erythrocytes of group A and a suspension of test erythrocytes of group B; and
- the aforementioned containers containing an anti-antigen blood-group test antibody distinctly contain an anti-A antibody and an anti-B antibody.

**41.** A kit according to claim 40 for blood phenotyping, including the Simonin test, of a sample of total blood from an individual, **characterized in that** it includes in addition containers distinctly containing test antibodies specific for the blood-group antigens, other than antigen A and B, whose presence or absence on the erythrocytes of the sample to be phenotyped is sought to be determined.

**42.** A kit according to one of the claims 39 to 41, **characterized in that** the aforementioned anti-antigen blood-group test antibodies contained in the aforementioned containers are in desiccated form.

**43.** A kit according to one of the claims 39 to 42, **characterized in that** the aforementioned containers are microplate wells.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 0194212 A
- EP 0755719 A
- EP 0058780 A
- WO 9802752 A
- WO 9217781 A
- EP 0426170 A
- EP 0351857 A
- EP 0528708 A
- EP 0230768 A
- EP 0348191 A
- WO 0240159 A
- WO 0246758 A
- FR 2461521